# EUROPEAN PATENT SPECIFICATION

(11) **EP 2 941 208 B1**
(45) Date of publication and mention of the grant of the patent: **24.07.2024**
(21) Application number: 14735280.1
(22) Date of filing: 07.01.2014
(51) Int. Cl.: A61B 17/16, B23B 45/00, A61B 17/17, B23B 39/14

(54) **FLEXIBLE DRILL BIT AND ANGLED DRILL GUIDE FOR USE WITH THE SAME**
FLEXIBLER BOHRMEISSEL UND ABGEWINKELTE BOHRFÜHRUNG ZUR VERWENDUNG DAMIT
TRÉPAN FLEXIBLE ET GUIDE DE FORAGE OBLIQUE DESTINÉ À ÊTRE UTILISÉ AVEC CE DERNIER

(30) Priority: 07.01.2013 US 201313735806; 11.02.2013 US 201313764565; 23.04.2013 US 201361815074 P; 04.11.2013 US 201361899419 P
(43) Date of publication of application: 11.11.2015
(73) Proprietor: Stryker Puerto Rico, LLC, Puerto Rico 00901 (PR)
(72) Inventor: BURLEY, J., Brook, Mountain View, CA 94041 (US); LANTZ, Andrew, Redwood City, CA 94063 (US); GRAUL, Jeremy, Elk Grove, CA 95624 (US); PAGE, Brett, M., Sunnyvale, CA 94086 (US); FLOM, James, San Carlos, CA 94070 (US); PANDYA, Sudip, Fremont, CA 94538 (US); PAMICHEV, Chris, Cupertino, CA 95014 (US)
(74) Representative: V.O.
(86) International application number: PCT/US2014/010511
(87) International publication number: WO 2014/107729

(56) References cited:
- EP-A2- 1 584 299
- WO-A1-2011/161676
- US-A- 4 541 423
- US-A- 4 541 423
- US-A- 5 387 218
- US-A- 5 601 550
- US-A1- 2005 059 975
- US-A1- 2007 264 093
- US-A1- 2007 264 093
- US-A1- 2009 149 890
- US-A1- 2010 191 248
- US-A1- 2010 191 248
- US-A1- 2010 286 694
- US-A1- 2010 292 722
- US-A1- 2011 015 674
- US-A1- 2011 015 674
- US-A1- 2011 251 621
- US-A1- 2012 123 417
- US-B1- 6 309 396
- US-B1- 6 309 396

## Description

### Field Of The Invention

This invention relates to surgical methods and apparatus in general, and more particularly to methods and apparatus for drilling a hole in bone.

### Background Of The Invention

### The General Trend Toward Treating Joint Pathologies Using Minimally-Invasive, And Earlier, Interventions

The current trend in orthopedic surgery is to treat joint pathologies using minimally-invasive techniques. Such minimally-invasive, "keyhole" surgeries generally offer numerous advantages over traditional, "open" surgeries, including reduced trauma to tissue, less pain for the patient, faster recuperation times, etc.

By way of example but not limitation, it is common to re-attach ligaments in the shoulder joint using minimally-invasive, "keyhole" techniques which do not require laying open the capsule of the shoulder joint. By way of further example but not limitation, it is also common to repair torn meniscal cartilage in the knee joint, and/or to replace ruptured ACL ligaments in the knee joint, using minimally-invasive, keyhole techniques.

While such minimally-invasive approaches can require additional training on the part of the surgeon, such procedures generally offer substantial advantages for the patient and have now become standard procedures for treating many shoulder joint and knee joint pathologies.

In addition to the foregoing, in view of the inherent advantages and widespread availability of minimally-invasive approaches for treating pathologies of the shoulder joint and the knee joint, the current trend is to provide such treatment much earlier in the lifecycle of the pathology, so as to address patient pain and so as to reduce the likelihood of exacerbating the pathology itself. This is in marked contrast to traditional surgical practices, which generally dictated postponing surgical procedures for as long as possible so as to spare the patient from the substantial trauma generally associated with invasive surgery.

### Treatment For Pathologies Of The Hip Joint

Unfortunately, minimally-invasive treatments for pathologies of the hip joint have lagged far behind minimally-invasive treatments for pathologies of the shoulder joint and the knee joint. This is generally due to (i) the complex geometry of the hip joint itself, and (ii) the nature and location of the pathologies which are typically encountered in the hip joint.

More particularly, the hip joint is generally considered to be a "tight" joint, in the sense that there is relatively little room to maneuver within the confines of the joint itself. This is in marked contrast to the shoulder joint and the knee joint, which are generally considered to be relatively "spacious" joints (at least when compared to the hip joint). As a result, it is generally relatively difficult for surgeons to perform minimally-invasive procedures on the hip joint.

Furthermore, the pathways and approaches for entering the interior of the hip joint (i.e., the natural pathways which exist between adjacent bones and/or delicate neurovascular structures) are generally much more limited for the hip joint than for the shoulder joint or the knee joint. This limited access further complicates a surgeon's ability to effectively perform minimally-invasive procedures on the hip joint.

In addition to the foregoing, the nature and location of the pathologies of the hip joint also complicate a surgeon's ability to perform minimally-invasive procedures on the hip joint. By way of example but not limitation, consider a typical labrum tear or detachment in the hip joint. In this situation, instruments must generally be introduced into the joint space at an angle of approach which is offset from the angle at which the instrument addresses the joint anatomy. This makes drilling into bone, for example, a significantly more complicated procedure than in a case where the angle of approach is effectively aligned with the angle at which the instrument addresses the joint anatomy, such as is frequently the case in the shoulder joint. Furthermore, since the working space within the hip joint is typically extremely limited, it is even more difficult to properly adjust the alignment of surgical instruments (e.g., a drill) where the angle of approach is not aligned with the optimal angle for the instrument to address the joint anatomy.

As a result of the foregoing, minimally-invasive hip joint procedures are still relatively difficult to perform and hence less common in practice. Consequently, patients are typically forced to manage and endure their hip pain for as long as possible, until a resurfacing procedure or a partial or total hip replacement procedure can no longer be avoided. These resurfacing or replacement procedures are generally then performed as a highly-invasive, open procedure, replete with all of the disadvantages associated with highly-invasive, open procedures.

As a result, there is, in general, a pressing need for improved methods and apparatus for treating pathologies of the hip joint.

More particularly, there is a pressing need for improved methods and apparatus for introducing instruments into the joint space where the instruments will address the joint anatomy at an angle which is offset from the angle of approach. By way of example but not limitation, in some cases it may be desirable to drill into bone at an angle which is offset from the angle at which the drill is inserted into the joint space, in order to create a hole in the bone at an optimum location, e.g., at an optimum location to receive a suture anchor for use in effecting a labral repair.

US2011/0015674A1 relates to an apparatus for inserting a suture anchor into an internal anatomical site through a cannula, or percutaneously, has a curved hollow guide for insertion into the cannula and/or tissue. The guide has a proximal end and a distal end. The distal end has a "parabolic " shape for engaging the intended anatomical site such as a glenoid. A flexible obturator is insertable through the curved guide from the proximal end to the distal end to facilitate insertion of the guide through the cannula. A flexible drill is insertable through the curved guide from the proximal to the distal end once the guide is in place. A flexible inserter is provided for inserting a suture anchor into a bore at the anatomical site formed by the flexible drill.

US2010/0191248A1 relates to a drill guide assembly for drilling a tunnel having a fixed, non-zero radius of curvature, where the drill guide assembly includes a housing and a sleeve, or cutting tube, configured to reciprocate within the distal portion where the sleeve, or cutting tube, is configured to receive a bone cutting instrument.

US4,541,423 relates to a drilling apparatus for attachment to a rotary motor including a flexible shaft confined in an elongated tubular sheath. The sheath is formed from a semi-rigid material which is bendable to a desired curvature, at the use site to select the curvature of the drilled hole, and which is rigid enough to retain that curvature in use. A drilling bit is fixed to one end of the flexible shaft, and includes a shank coacting with the distal end of the sheath to rotationally guide the drilling bit. The flexible shaft projects from the sheath at the proximal end so that both components may be secured to a drilling motor which is manipulated to guide the sheath while rotating the cutting bit. The sheath may remain in the drilled hole temporarily as a liner to guide the passage therethrough of a relatively stiff wire or other filamentary member.

US2007/0264093A1 relates to a flexible, extendable extension assembly used to create extended length holes and holes through inaccessible areas in walls and framing. The extension assembly including a flexible rod having first and second ends; and a holding means secured to the first end of the rod for releasably holding a tool bit for rotation with the rod.

US2010/0286694A1 relates to a surgical drilling instrument with a Curved Burr Attachment having an adapter removably coupled to a drill motor and a burr attachment coupled to said adapter. The burr attachment includes a tube with a wire shaft supported therein and a coil wire surrounding said wire shaft and having each of the coils touch the outer periphery surface of the wire shaft and the inner peripheral surface of said tube to enhance cooling of said outer periphery of said tube from the heat generated by said wire shaft and attenuate vibrations while allowing said burr attachment to be bent without kinking. The burr attachment includes a proximal end support and a distal end support formed on the end of said burr attachment wherein the wire shaft is affixed to said proximal end support and rotates relative to said distal end support and includes detents formed on each of the supports for engaging balls in said adapter so as to be operatively connected thereto when the burr attachment is aligned in said adapter to couple the burr attachment to said adapter for rotary motion of said wire shaft and locating the cutter of said burr attachment to a precise location relative to the end of the adapter. The burr attachment being disposable and the adapter being re-usable.

### Summary of the Present Invention

These and other objects of the present invention are addressed by the provision of a surgical apparatus according to claim 1

### Brief Description Of The Drawings

These and other objects and features of the present invention will be more fully disclosed or rendered obvious by the following detailed description of the preferred embodiments of the invention, which is to be considered together with the accompanying drawings wherein like numbers refer to like parts and further wherein:
Fig. 1 is a schematic view showing a flexible drill bit formed in accordance with the present invention;
Figs. 2-5 are schematic views showing the flexible drill bit of Fig. 1 being used in conjunction with a curved drill guide to form a hole in bone;
Figs. 6 and 7 are schematic views showing another flexible drill bit formed in accordance with the present invention and being used in conjunction with a curved drill guide to form a hole in bone;
Fig. 8 is a schematic view showing still another flexible drill bit formed in accordance with the present invention;
Fig. 9 is a schematic view showing the flexible drill bit of Fig. 8 being used in conjunction with a curved drill guide to form a hole in bone;
Fig. 10 is a schematic view showing the flexible drill bit of Fig. 1 with a helical coil disposed over a portion of the flexible drill bit;
Fig. 11 is a schematic view showing the flexible drill bit of Fig. 1 with another form of helical coil disposed over a portion of the flexible drill bit;
Fig. 12 is a schematic view showing the flexible drill bit of Fig. 1 with an over-molded sheath disposed over a portion of the flexible drill bit;
Fig. 13 is a schematic view showing the flexible drill bit of Fig. 1 with a metal braid or mesh disposed over a portion of the flexible drill bit; a
Fig. 14 is a schematic view showing a form of flexible drill bit not according to claim 1;
Fig. 15 is a schematic view showing still another form of flexible drill bit not according to claim 1;
Figs. 16-19 are schematic cross-sectional views taken along lines A-A, B-B, C-C and D-D, respectively, of Fig. 15;
Figs. 20-23 are schematic cross-sectional views taken along lines A-A, B-B, C-C and D-D, respectively, of Fig. 15 ;
Fig. 24 is a schematic view showing another form of flexible drill bit not according to claim 1;
Fig. 25 is a schematic cross-sectional view taken along line A-A of Fig. 24;
Fig. 26 is a schematic view showing still another form of flexible drill bit not according to claim 1;
Fig. 27 is an enlarged schematic view showing selected portions of the flexible drill bit of Fig. 26;
Figs. 28-32 are schematic views showing various forms of cutting tips which may be used with the flexible drill bit of the present invention;
Figs. 33-36 are schematic views showing a novel angled drill guide formed in accordance with the present invention;
Fig. 37 is a schematic view showing how a flexible drill bit exiting the distal end of an angled drill guide will tend to exit the angled drill guide with an off-centered disposition;
Fig. 38 is a schematic view showing how an angled drill guide may be provided with a dimple so as to re-center the flexible drill bit as it exits the distal end of the angled drill guide;
Figs. 39 and 40 are schematic views showing how an angled drill guide may be provided with a tapered inner lumen so as to re-center the flexible drill bit as it exits the distal end of the angled drill guide;
Fig. 41 is a schematic view showing how an angled drill guide may be provided with compound curves so as to re-center the flexible drill bit as it exits the distal end of the angled drill guide;
Fig. 42 is a schematic view showing how an angled drill guide may be provided with a novel handle so as to facilitate pushing the distal end of the angled drill guide directly against the outer surface of the material (e.g., bone) which is to be drilled, whereby to provide more stable drilling;
Figs. 43 and 44 are schematic views showing a novel articulating angled drill guide formed in accordance with the present invention;
Figs. 45 and 46 are schematic views showing another novel articulating angled drill guide formed in accordance with the present invention;
Figs. 47-49 and 49A are schematic views showing still another novel articulating angled drill guide formed in accordance with the present invention;
Figs. 50-57 are schematic views showing how an articulating angled drill guide and flexible drill bit may be used to drill a hole in a surface of a joint;
Figs. 58-60 are schematic views showing a novel friction-reducing flexible drill bit not according to claim 1
Figs. 60A-60E are schematic views showing a novel flexible drill bit formed not according to claim 1, and a novel angled drill guide ; and
Figs. 61-66 are schematic views showing another novel angled drill guide which may be used with a flexible drill bit.

### Detailed Description Of The Preferred Embodiments

### Flexible Drill Bit Having A "Unibody" Construction

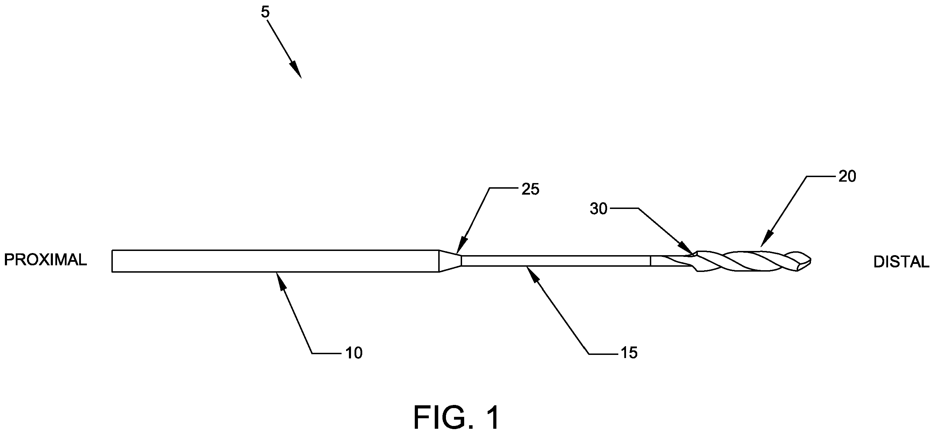

Looking first at Fig. 1, there is a shown a flexible drill bit 5 formed in accordance with the present invention. Flexible drill bit 5 comprises three sections, i.e., a full diameter shaft portion 10, a reduced diameter shaft portion 15, and a fluted cutting tip portion 20. Full diameter shaft portion 10, reduced diameter shaft portion 15, and fluted cutting tip portion 20 are all formed integral with one another so as to create a flexible drill bit having a "unibody" construction. If desired, a transition area 25 may be formed between full diameter shaft portion 10 and reduced diameter shaft portion 15, and/or a transition area 30 may be formed between reduced diameter shaft portion 15 and fluted cutting tip portion 20.

The "unibody" construction eliminates the need for a mechanical joint connecting the cutting tip of the flexible drill bit (e.g., fluted cutting tip portion 20) to the flexible portion of the flexible drill bit (e.g., reduced diameter shaft portion 15), thereby eliminating a possible point of failure. Such a failure of a mechanical joint can be particularly problematic if the mechanical joint were to fail below the surface of the bone (i.e., subchondral); in this scenario, it would be unlikely that the portion of the drill bit left in the bone could be recovered. Thus, the possible failure of such a mechanical joint creates a serious clinical concern. In addition, the "unibody" construction eliminates the need for a mechanical joint connecting the flexible portion of the flexible drill bit (e.g., reduced diameter shaft portion 15) to the full diameter shaft portion (e.g., full diameter shaft portion 10) of the flexible drill bit, thus eliminating another possible point of failure.

The flexible drill bit may comprise a material such as Nitinol, stainless steel, titanium, or other appropriate material, but is preferably Nitinol.

The reduced diameter shaft portion 15 of flexible drill bit 5 provides flexibility in that portion of the drill bit while still providing the torsional strength needed to drill into bone. The diameter of the reduced diameter shaft portion 15 is preferably approximately 20-40% smaller than the diameter of the full diameter shaft portion 10, and more preferably approximately 25% smaller than the diameter of the full diameter shaft portion 10. In an alternative embodiment, the diameter of the reduced diameter shaft portion 15 is preferably approximately 5-25% smaller than the diameter of the fluted cutting tip portion 20, and more preferably approximately 15% smaller than the diameter of the fluted cutting tip portion 20.

The transition area 30 located between fluted cutting tip portion 20 and the reduced diameter shaft portion 15, and/or the transition area 25 located between the reduced diameter shaft portion 15 and the full diameter shaft portion 10, are preferably formed so as to distribute stress, whereby to minimize the possibility of mechanical failure at the transition areas.

Full diameter shaft portion 10 provides a region, preferably at its proximal end, in which flexible drill bit 5 can be attached to a drill.

Fluted cutting tip portion 20 is preferably sufficiently rigid to form a straight hole in the target bone. To that end, the length of fluted cutting tip portion 20 must be short enough so that the fluted cutting tip portion 20 may pass through the curve of a curved drill guide or curved cannula. In one preferred embodiment, fluted cutting tip portion 20 has a length which is approximately 6 times greater than its diameter.

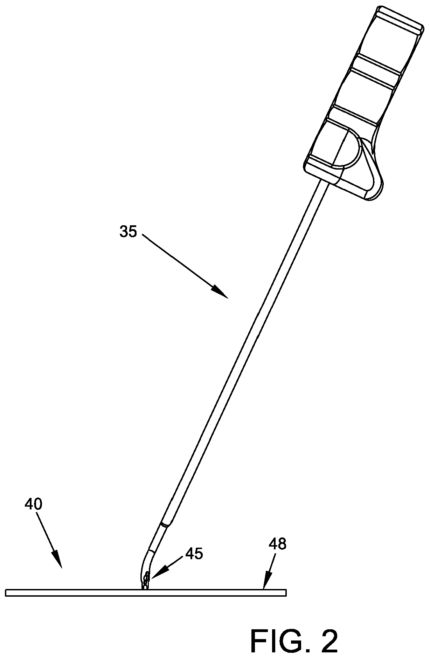

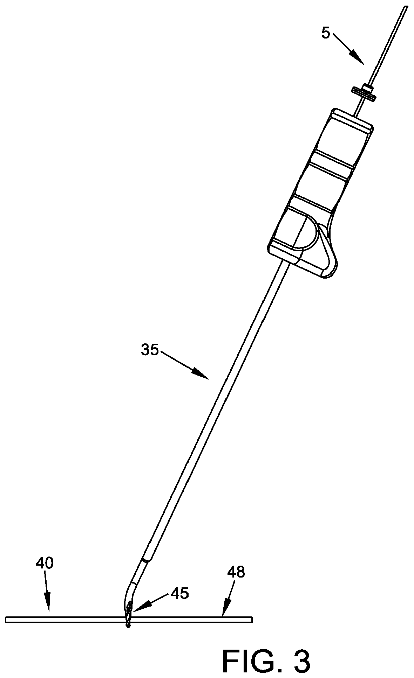

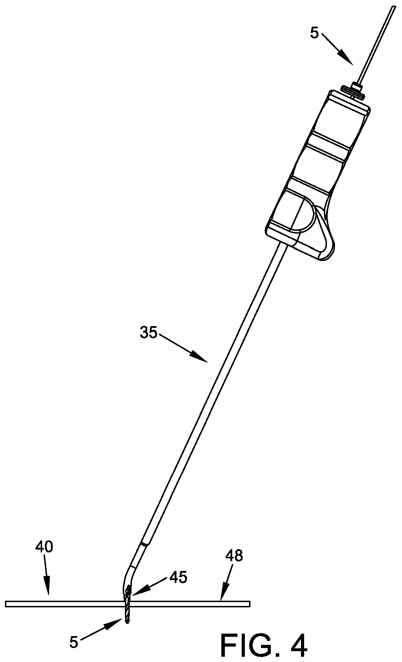

Figs. 2-5 show flexible drill bit 5 being used in conjunction with a curved drill guide 35 to form a hole in a bone 40. More particularly, as seen in the figures, the distal tip 45 of curved drill guide 35 is placed against the outer surface 48 of bone 40, and then flexible drill bit 5 is passed through the lumen 50 of curved drill guide 35 and directed into bone 40 so as to make the hole in the bone at the desired location and with the desired angle.

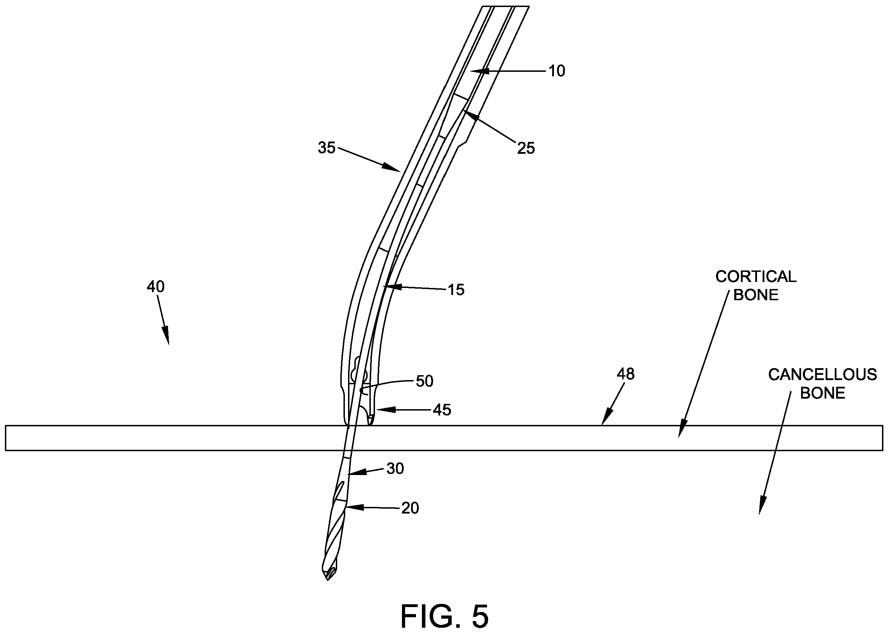

Note in Fig. 5 how the curvature of curved drill guide 35 can combine with the differences in the diameters of the reduced diameter shaft portion 15 and lumen 50 so as to result in a non-perpendicular entry of flexible drill bit 5 into the bone, even where distal tip 45 of curved drill guide 35 is disposed substantially perpendicular to outer surface 48 of the bone. In other words, the curvature of curved drill guide 35 can combine with the differences in the diameters of reduced diameter shaft portion 15 and lumen 50 so that fluted cutting tip portion 20 is not perfectly coaxial with lumen 50 as fluted cutting tip portion 20 emerges from the distal end of curved drill guide 35. It will be apparent to one skilled in the art that, depending on the bone surface contour and/or the angle of approach of curved drill guide 35, the curved drill guide 35 may not always be disposed perpendicular to outer surface 48 of the bone. In this scenario, it is typically still desirable to have the fluted cutting tip portion 20 centered and aligned with the end of the curved drill guide 35.

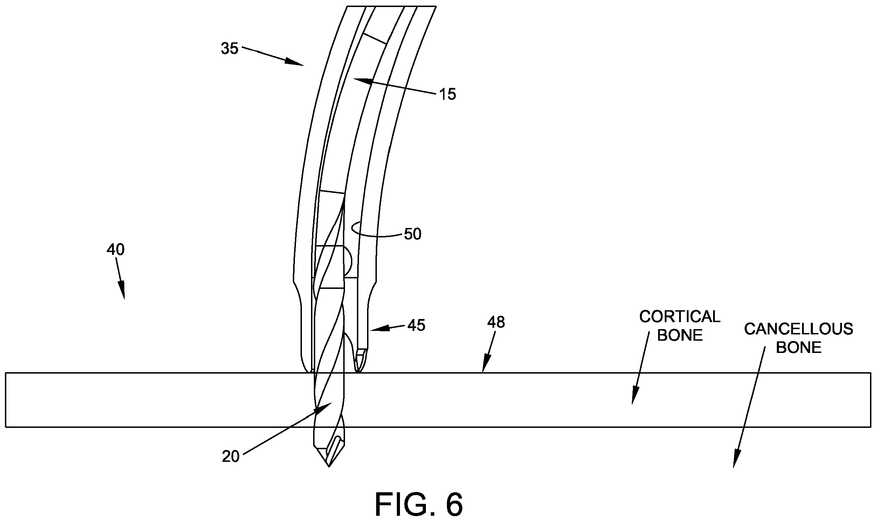

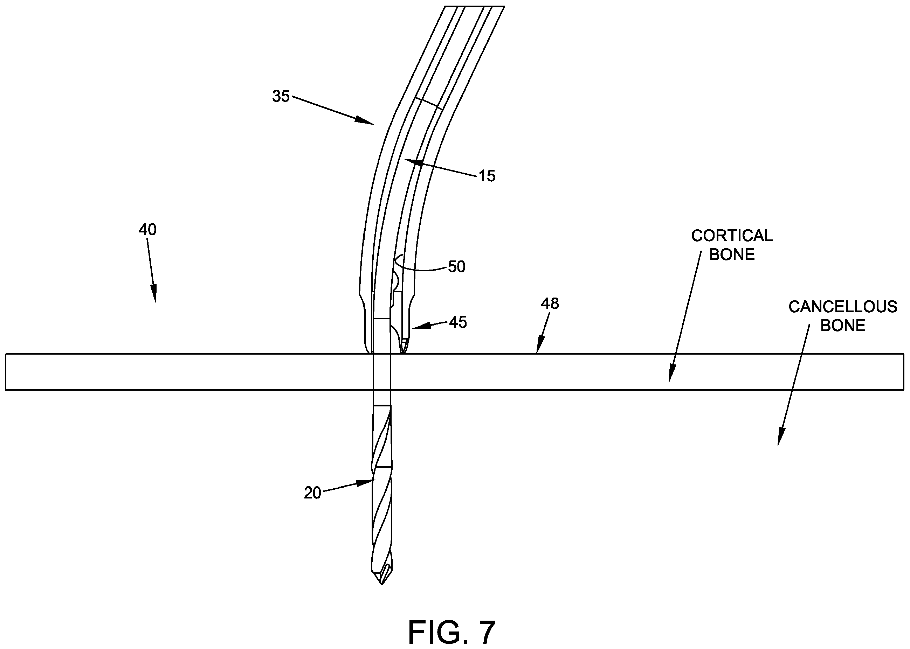

Figs. 6 and 7 show another form of the invention where the diameter of reduced diameter shaft portion 15 is sized so as to be closer to the diameter of fluted cutting tip portion 20 and so as to be somewhat closer to the diameter of lumen 50 of curved drill guide 35. In this form of the invention, flexible drill bit 5 will tend to enter the bone closer to perpendicular. In other words, in this form of the invention, fluted cutting tip portion 20 will tend to remain more coaxial with lumen 50 as fluted cutting tip portion 20 emerges from the distal end of curved drill guide 35.

In one preferred form of the invention, full diameter shaft portion 10 has a length of approximately 12 inches and a diameter of approximately 0.063 inch; reduced diameter shaft portion 15 has a length of approximately 1.5 inches and a diameter of approximately 0.047 inch; fluted cutting tip portion 20 has a total fluted length of approximately 0.5 inch, of which approximately 0.325 inch is of constant outer diameter (OD) of approximately 0.055 inch and the remaining length of 0.175 inch tapers on the proximal end of the flutes; and curved drill guide 35 has a radius of curvature of approximately 1.25 inches, a curve of approximately 25 degrees, and a lumen 50 diameter of approximately 0.071 inch. In this preferred form of the invention, flexible drill bit 5 is capable of transmitting at least approximately 2 in-lbs (inch-pounds) of torque without failure, and more preferably approximately 3 in-lbs (inch-pounds) of torque without failure. In this configuration, fluted cutting tip portion 20 can pass through lumen 50 of curved drill guide 35. Specifically, fluted cutting tip portion 20 is substantially rigid due to its larger diameter (i.e., as compared to reduced diameter shaft portion 15); however, there is sufficient clearance between the outer diameter of fluted cutting tip portion 20 and lumen 50 so that fluted cutting tip portion 20 passes through lumen 50 without significant interference. The length of the fluted cutting tip portion 20 is preferably less than the depth of the hole which it will be used to drill. In other words, when the flexible drill bit 5 is used to form a bone hole, the entire length of the fluted cutting tip portion 20 will pass into the bone along with a portion of the reduced diameter shaft portion 15.

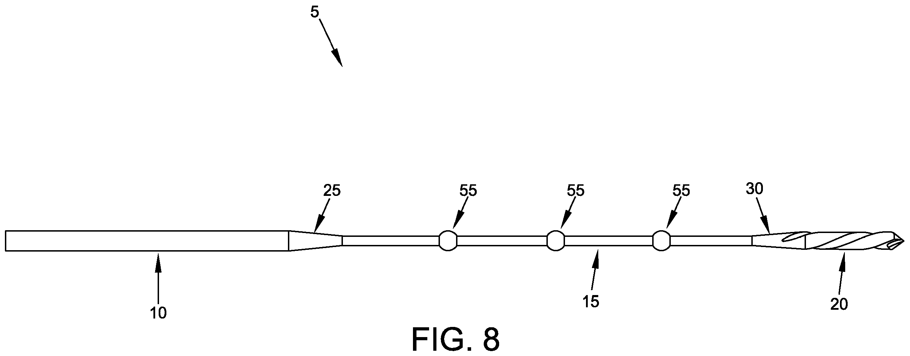

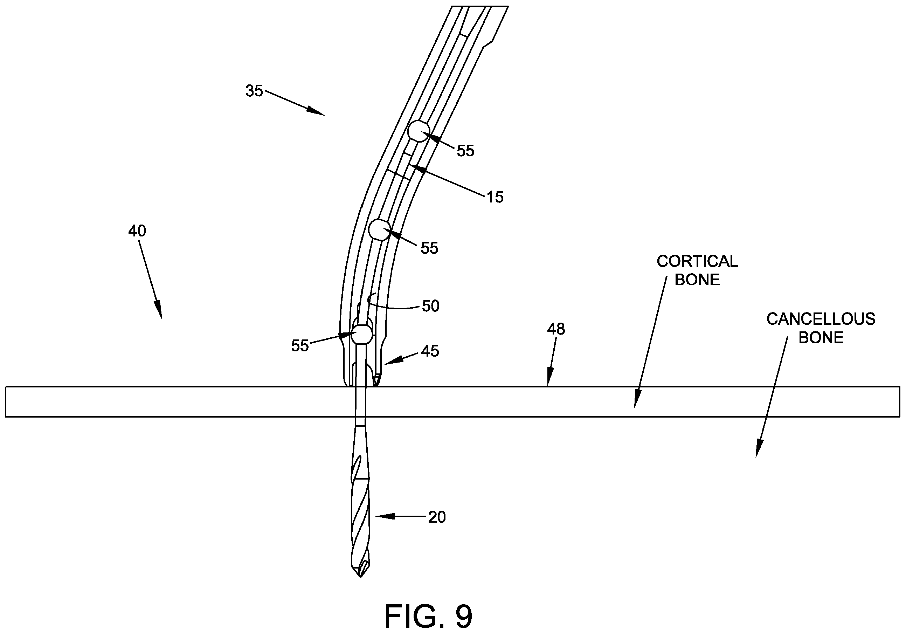

In another form of the invention, and looking now at Figs. 8 and 9, one or more enlargements 55 may be formed on the reduced diameter shaft portion 15 of flexible drill bit 5. Enlargements 55 preferably have an outer diameter similar to the outer diameter of full diameter shaft portion 10 and thus serve to keep flexible drill bit 5 centered in lumen 50 of curved drill guide 35 even where reduced diameter shaft portion 15 has a diameter which is significantly less than the diameter of lumen 50 of curved drill guide 35. In this form of the invention, enlargements 55 will also keep flexible drill bit 5 closer to perpendicular as it enters bone 40. In other words, in this form of the invention, fluted cutting tip portion 20 will tend to remain more coaxial with lumen 50 as fluted cutting tip portion 20 emerges from the distal end of curved drill guide 35. In another embodiment, enlargements 55 have an outer diameter similar to the outer diameter of fluted cutting tip portion 20.

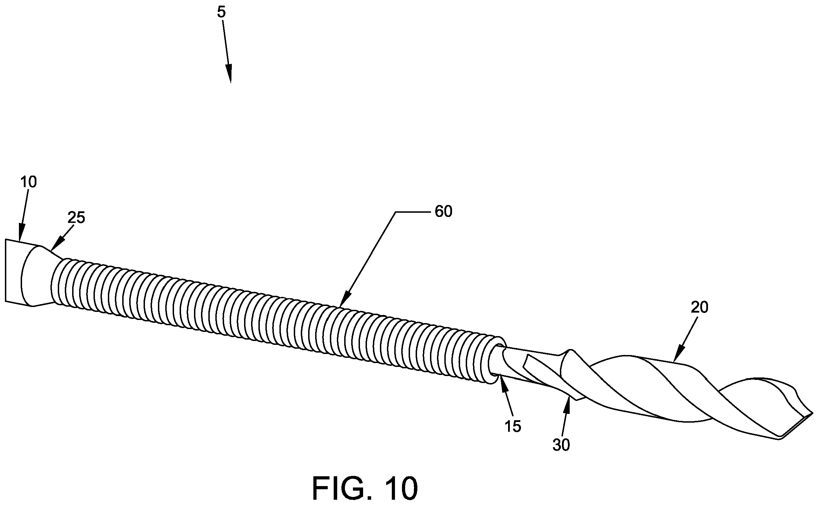

In another embodiment, and looking now at Fig. 10, a helical coil 60 may be positioned over reduced diameter shaft portion 15 of flexible drill bit 5 so as to supplement the torque needed to drill into bone while still providing the flexibility needed to maneuver around a curve in a curved drill guide (e.g., curved drill guide 35) or curved cannula. Helical coil 60 also helps to keep flexible drill bit 5 centered in a curved drill guide (e.g., curved drill guide 35) and reduce the "mismatch" angle between flexible drill bit 5 and the end of curved drill guide 35.

More particularly, helical coil 60 provides additional torsional strength and increased diameter to the reduced diameter shaft portion 15 of flexible drill bit 5 without significantly reducing the flexibility of the drill bit. The increased diameter of reduced diameter shaft portion 15 of flexible drill bit 5 (due to the presence of helical coil 60) creates a close fit within the curved drill guide or curved cannula, thereby ensuring that the drill bit remains coaxial with the curved drill guide or curved cannula as the flexible drill bit emerges from the distal end of the curved drill guide or curved cannula and engages the bone (or other material) which is being drilled.

Helical coil 60 may form a close fit around reduced diameter shaft portion 15 and be sized so that it rests between transition area 25 and transition area 30. Helical coil 60 may be resilient and may be stretched slightly (in its diameter) from its unbiased condition so as to allow the helical coil to be positioned onto reduced diameter shaft portion 15; in other words, in a free condition, the helical coil 60 has an inner diameter which is smaller than the outer diameter of the reduced diameter shaft portion 15. Helical coil 60 may simply sit on reduced diameter shaft portion 15, or it may be secured to reduced diameter shaft portion 15 (e.g., at one end of helical coil 60, at both ends of helical coil 60, and/or intermediate helical coil 60, etc.). In one preferred embodiment, helical coil 60 is secured at both its ends to reduced diameter shaft portion 15 and forms a close fit with reduced diameter shaft portion 15 or is stretched slightly diametrically from its unbiased condition and then set onto reduced diameter shaft portion 15. Helical coil 60 may be secured to reduced diameter shaft portion 15 by soldering, adhesive, welding, mechanical interlock, or other appropriate attachment means. Helical coil 60 is preferably formed and positioned so that when the flexible drill bit is used to drill into bone, the helical coil will tighten onto reduced diameter shaft portion 15 during drilling. For example, if a flexible drill bit 5 rotates in a clockwise direction (when viewed from proximal to distal), the helical coil should have a counter-clockwise winding direction (again, when viewed from proximal to distal). This arrangement provides a preferred transfer of torque between reduced diameter shaft portion 15 and helical coil 60; in other words, reduced diameter shaft portion 15 and helical coil 60 share torque transmission between full diameter shaft portion 10 and fluted cutting tip portion 20.

Helical coil 60 may comprise a material such as stainless steel, Nitinol or other suitable material. Helical coil 60 may comprise a wire of round or rectangular cross-section. Although Fig. 10 depicts a closely wound helical coil (i.e., with substantially no space between the coils), an alternative embodiment comprises spacing between the coils.

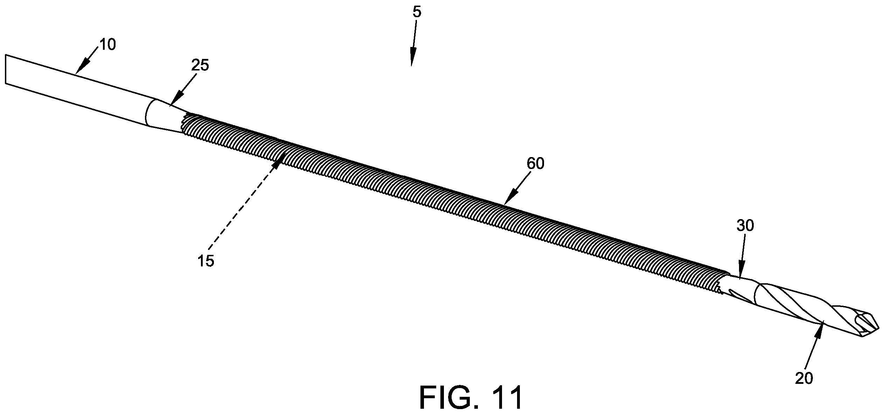

Fig. 11 shows a construction similar to that of Fig. 10, except that helical coil 60 comprises a multi-strand coil (i.e., multiple strands are coiled together). In this embodiment, adjacent multiple strands follow the same coil pitch. However, even with coils touching each other, the pitch can be greater than a single strand arrangement (e.g., as shown in Fig. 10). This construction (i.e., larger pitch with coils touching) can be beneficial to reduce "play" in the coil; that is, as the flexible drill bit 5 starts drilling into bone, the helical coil 60 will more quickly respond in carrying a portion of the torque.

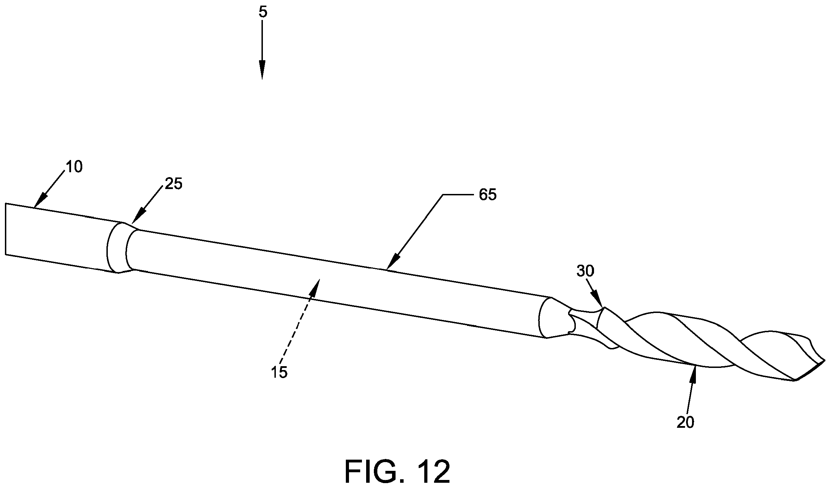

In another embodiment, and looking now at Fig. 12, an over-molded sheath 65 may be positioned over reduced diameter shaft portion 15 of flexible drill bit 5. Over-molded sheath 65 provides reduced friction (e.g., with curved drill guide 35 and/or bone 40) and increased diameter to reduced diameter shaft portion 15 of flexible drill bit 5, while still enabling bending of the reduced diameter shaft portion 15 of flexible drill bit 5. Over-molded sheath 65 may comprise a low-friction polymer such as Nylon or polytetrafluoroethylene (PTFE). Over-molded sheath 65 may be over-molded onto reduced diameter shaft portion 15 by injection molding or by diameter reduction (e.g., by shrinking or melting over-molded sheath 65 onto reduced diameter shaft portion 15).

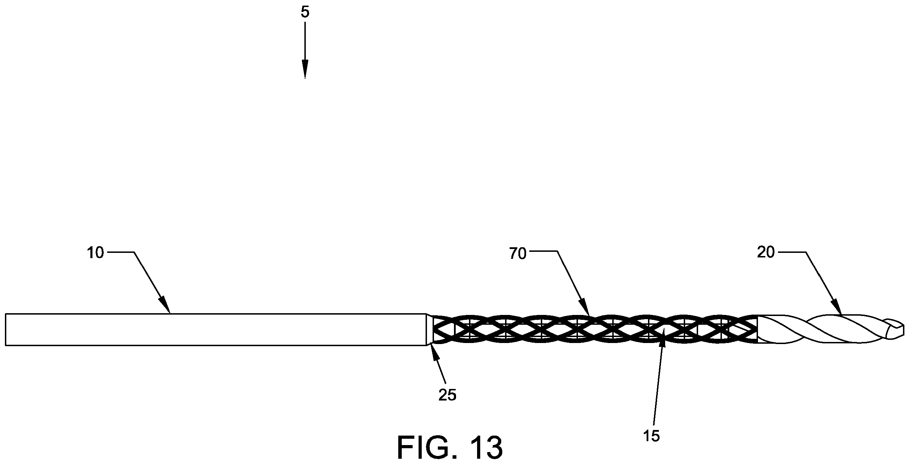

In another embodiment, and looking now at Fig. 13, a braid or mesh 70 (preferably but not necessarily formed out of metal) may be positioned over reduced diameter shaft portion 15 of flexible drill bit 5. Metal braid or mesh 70 provides torsional strength and increased diameter to reduced diameter shaft portion 15 of flexible drill bit 5, while still enabling bending/flexing of reduced diameter shaft portion 15 of flexible drill bit 5. Metal braid or mesh 70 may comprise a material such as stainless steel or Nitinol. It may comprise wire having a rectangular cross-section. Metal braid or mesh 70 may be attached to reduced diameter shaft portion 15 of flexible drill bit 5 by attaching one or both of its ends to the reduced diameter shaft portion, or by attaching an intermediate portion of metal braid or mesh 70 to reduced diameter shaft portion 15, or both (e.g., by welding, adhesive, etc.). Alternatively, or additionally, a polymer (e.g., Pebax) may be heated and melted into the metal braid or mesh 70 so as to create a solid structure atop reduced diameter shaft portion 15. This polymer can provide a lower friction surface than the metal braid or mesh 70 alone, and can provide some torque transmission as well.

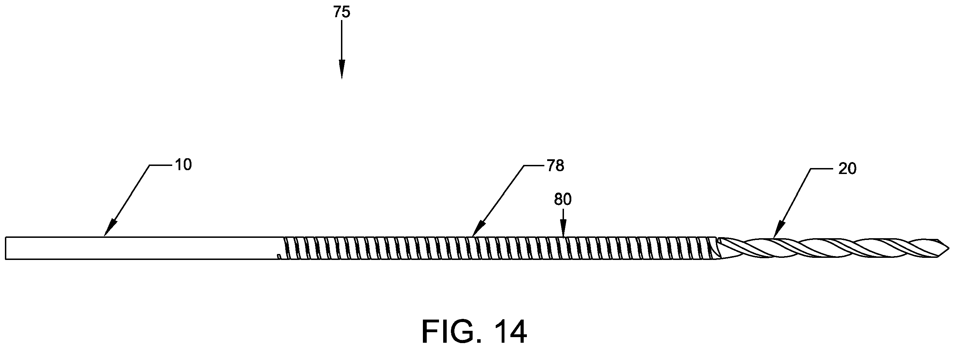

Looking next at Fig. 14, there is shown a flexible drill bit 75 which is similar to the flexible drill bit 5 shown in Fig. 1, but not according to claim 1. Instead of proving a reduced diameter shaft portion (e.g., the aforementioned reduced diameter shaft portion 15) between the full diameter shaft portion (e.g., the aforementioned full diameter shaft portion 10) and the fluted cutting tip portion (e.g., the aforementioned fluted cutting tip portion 20) in order to create the desired flexibility in the drill bit, the full diameter shaft portion extends all the way to the fluted cutting tip portion and portions of material are removed from the full diameter shaft portion so as to create the desired flexibility in the drill bit while providing greater torque-carrying strength as compared to simply a reduced-diameter shaft portion 15.

More particularly, in this embodiment, and looking now at Fig. 14, flexible drill bit 75 comprises a full diameter shaft portion 10 and a fluted cutting tip portion 20, with full diameter shaft portion 10 and fluted cutting tip portion 20 being formed integral with one another (i.e., a "unibody" design). In order to render the distal end 78 of full diameter shaft portion 10 flexible, material is removed from the outer surface of the full diameter shaft portion, but penetrating only a portion of the way through the full diameter shaft portion so as to leave an intact inner core, whereby to create a flexible portion along the full diameter shaft portion of the drill bit. In other words, the material is removed from the exterior of the full diameter shaft portion, but for only a portion of the radius of the full diameter shaft portion, so as to leave an intact inner core along this portion of the flexible drill bit. The material is removed in a pattern which enhances shaft flexibility but minimizes the reduction of torque transmission. In one preferred form , the material is removed in a spiral pattern as shown at 80 in Fig. 14 and may be accomplished by laser cutting, electrical discharge machining (i.e., EDM), machining, grinding or other means. For a clockwise rotating flexible drill bit 5, spiral cuts 80 are preferably formed in a clockwise pattern (when viewed from proximal to distal direction), but may also be formed in a counter-clockwise pattern.

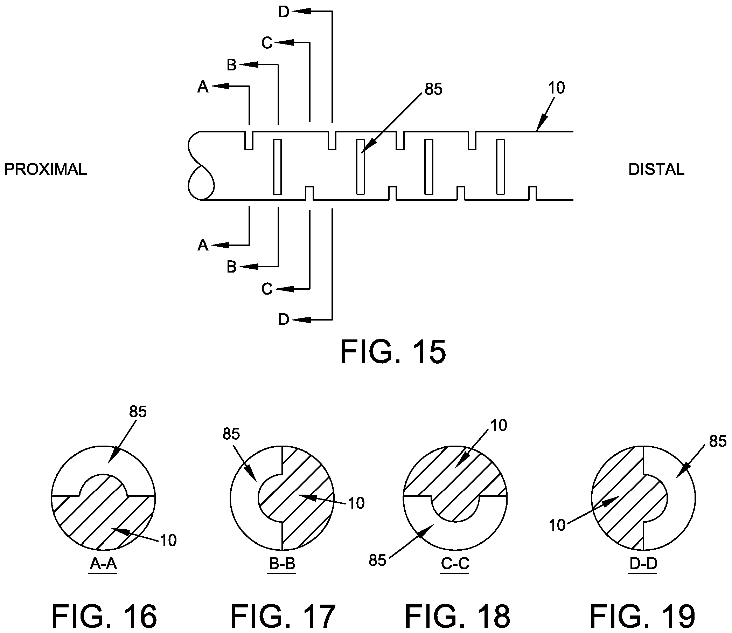

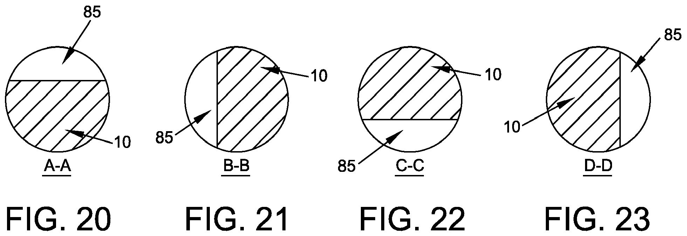

Material may also be removed from full diameter shaft portion 10 in other patterns so as to create a flexible, yet high torque transmitting, portion along the shaft of the drill bit. By way of example but not limitation, and looking now at Fig. 15, a series of transverse slots 85 (instead of the spiral cuts 80 shown in Fig. 14) may be cut into the shaft, with the slots preferably following a spiral or other geometric pattern. Transverse slots 85 may be formed with various configurations. Figs. 16-19 show one way of configuring transverse slots 85. Figs. 20-23 show another way of configuring transverse slots 85. Still other ways of configuring transverse slots 85 will be apparent to those skilled in the art in view of the present disclosure.

In this embodiment of the invention, flexible drill bit 75 may comprise a material such as stainless steel or Nitinol.

### Flexible Drill Bit Having A Multi-Body Construction

In another embodiment of the present invention, portions of the flexible drill bit (e.g., the cutting tip) may comprise separate components which are connected to the remaining portions of the flexible drill bit (e.g., the solid shaft) in order to provide a flexible drill bit having a multi-body construction.

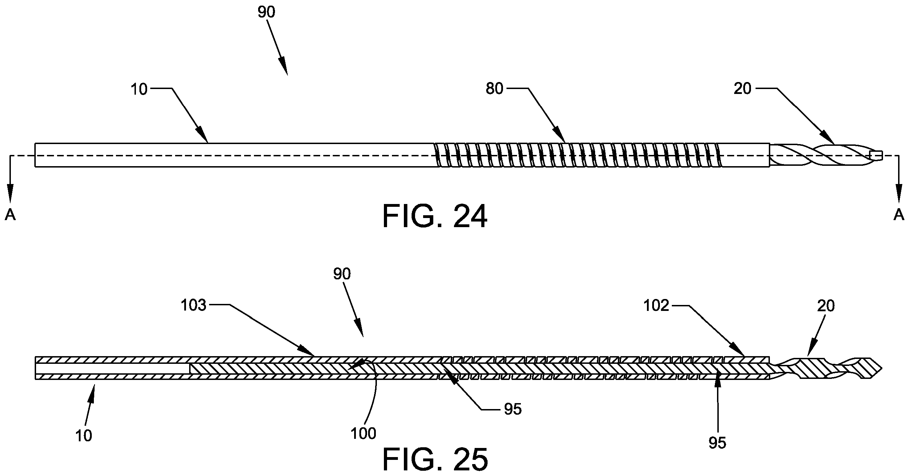

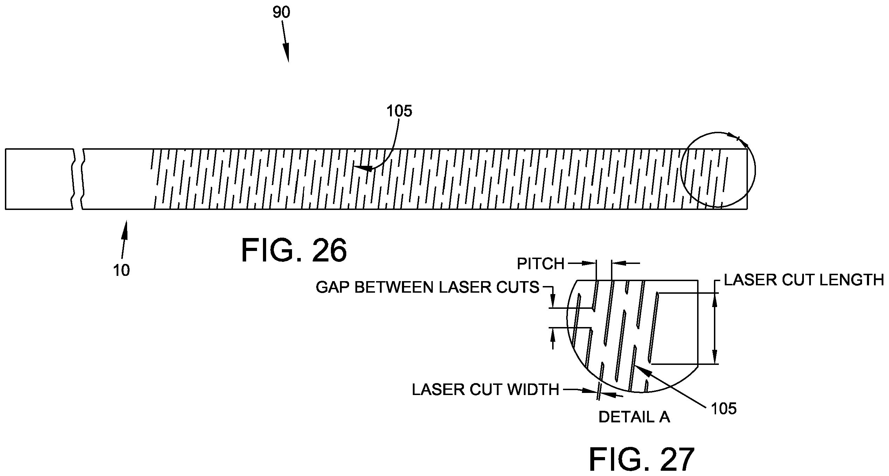

More particularly, and looking now at Figs. 24 and 25, there is a shown a flexible drill bit 90, not according to claim 1, comprising two components (i.e., full diameter shaft portion 10 and fluted cutting tip portion 20) which are connected together so as to form a flexible drill bit having three sections, i.e., a distal cutting tip, a proximal shaft and an intermediate flexible region. In this example, fluted cutting tip portion 20 comprises an elongated solid shaft 95 which is received within a lumen 100 formed in full diameter shaft portion 10 and then secured therein (e.g., by welding, adhesive bond, swaging, etc. or a combination thereof or other means well known in the art). Full diameter shaft portion 10 is preferably secured to fluted cutting tip portion 20 at the distal end of full diameter shaft portion 10, e.g., at 102. Flexible drill bit 90 may comprise additional points of securement between full diameter shaft portion 10 and fluted cutting tip portion 20 (e.g., proximal of the intermediate flexible region, such as at 103). The drill bit is rendered flexible by removing material from full diameter shaft portion 10, e.g., such as by forming spiral cuts 80 in full diameter shaft portion 10. Although spiral cuts 80 are shown in Figs. 24 and 25 as being formed in a clockwise pattern (when viewed from proximal to distal direction), they preferably would be formed in a counter-clockwise pattern when used with a clockwise-rotating drill (when viewed from proximal to distal) so that the spiral cuts would tend to tighten down on the elongated solid shaft 95 during drilling. Alternatively, and looking now at Figs. 26 and 27, the material may be removed as an interrupted spiral cut 105 so as to provide the desired flexibility to the drill bit. In one preferred form of this embodiment, the cuts are interrupted segment lengths of less than 120 degrees around the perimeter, have a opening - or width - which is less than the pitch distance (i.e., longitudinal distance between adjacent cuts), and have a gap between laser cuts which is approximately equal to the pitch distance. In one preferred form of this embodiment, the cuts have a slight angle relative to perpendicular to the longitudinal axis of the flexible drill bit 90.

Depending on the location(s) of securement between full diameter shaft portion 10 and fluted cutting tip portion 20 (e.g., at securement point 102, securement point 103, etc.), the torque may be transmitted through the intermediate flexible region by: (i) the full diameter shaft portion 10 (distal securement only); or (ii) through solid shaft 95 of fluted cutting tip portion 20 (proximal securement only); or (iii) shared between the two (both the proximal and distal securements).

### Cutting Tip Constructions

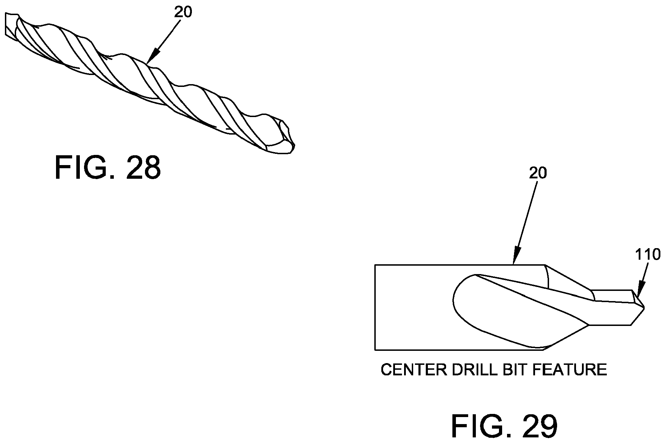

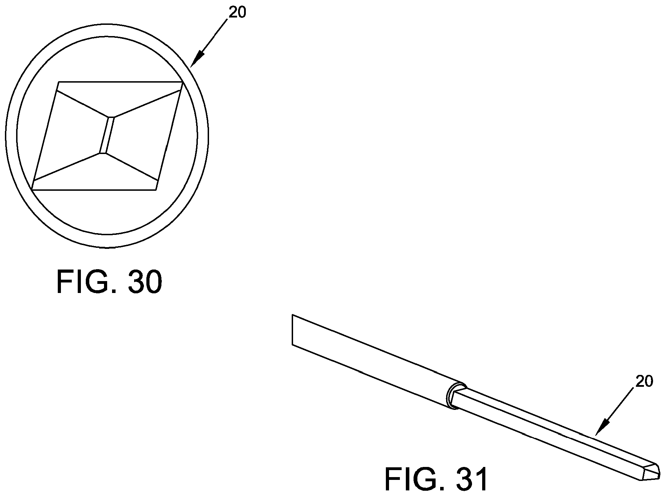

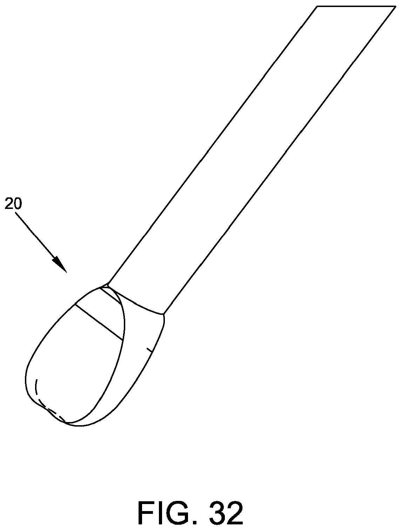

Looking now at Figs. 28-32, there are shown various shapes and designs of cutting tips which may be used in accordance with the present invention, e.g., a fluted cutting tip (Fig. 28), a fluted cutting tip with a centering feature 110 similar to a center drill bit (Fig. 29), a diamond shape (Figs. 30 and 31) or a forged or flattened tip (Fig. 32). Other cutting tip shapes and designs known in the art may also be used in accordance with the present invention (e.g., a bevel cut tip). In the cutting tip embodiment of a fluted cutting tip (e.g., Fig. 28), the inclusive angle at the tip may be approximately 30-120 degrees, is more preferably approximately 60-90 degrees, and is most preferably approximately 70 degrees.

### Helical Structures

In the foregoing disclosure, various constructions are provided in which the flexible drill bit comprises a helical structure. By way of example but not limitation, a helical coil 60 is mounted over reduced diameter shaft portion 15 (Figs. 10 and 11), a helical groove is formed in full diameter shaft portion 10 (Figs. 14 and 24-27), etc. These constructions are provided in order to maximize the flexibility of the drill bit while minimizing reduction of torque transmission capability through the drill bit. In this respect it will be appreciated that the configuration of the helical structure (i.e., the direction of the spiral) is preferably related to the direction of the applied torque, in order to maintain maximum torque transmission strength through the drill bit. However, the relationship of these may vary depending on the specific construction of the drill bit.

In the embodiment of a helical coil mounted over a reduced diameter shaft portion (Figs. 10 and 11), where the torque is intended to be applied in a clockwise direction (when viewed from the proximal end of the drill bit), it is preferred that the helix rotate counter-clockwise as it advances down the drill bit, and where the torque is intended to be applied in a counter-clockwise direction (when viewed from the proximal end of the drill bit), it is preferred that the helix rotate clockwise as it advances down the drill bit. Such an inverse relationship between the direction of the applied torque and the direction of the spiral will ensure that any deformation of the helical coil from the applied torque will cause the helical coil to tighten, whereby to preserve torque transmission through the helical coil.

In the embodiment of a helical groove formed in a full diameter shaft portion (Figs. 14 and 24-27), where the torque is intended to be applied in a clockwise direction (when viewed from the proximal end of the drill bit), it is preferred that the helix rotate counter-clockwise as it advances down the drill bit, and where the torque is intended to be applied in a counter-clockwise direction (when viewed from the proximal end of the drill bit), it is preferred that the helix rotate clockwise as it advances down the drill bit. The appropriate relationship between the direction of the applied torque and the direction of the spiral will maximize torque transmission while maintaining drill bit flexibility.

### General Construction

The flexible drill bit may comprise Nitinol or stainless steel or any other material which is flexible enough to bend into a curved state, and strong enough to transmit the torsional forces required for drilling into bone.

The entire shaft or portions of the shaft can be coated (e.g., with a biocompatible lubricant and/or a low-friction biocompatible outer sleeve such as a low-friction polymer, etc.) so as to reduce friction (e.g., with curved drill guide 35 and/or bone 40).

### Angled Drill Guide For Use With Flexible Drill Bit

In the preceding description, a flexible drill bit is disclosed for use in drilling a hole in material (e.g., bone) where the angle of approach is offset from the angle at which the drill is to enter the material.

In accordance with the present invention, there is now also provided a novel angled drill guide (i.e., a curved drill guide) used to guide entry of the flexible drill bit into the target material (e.g., bone) while the flexible drill bit is in its curved configuration.

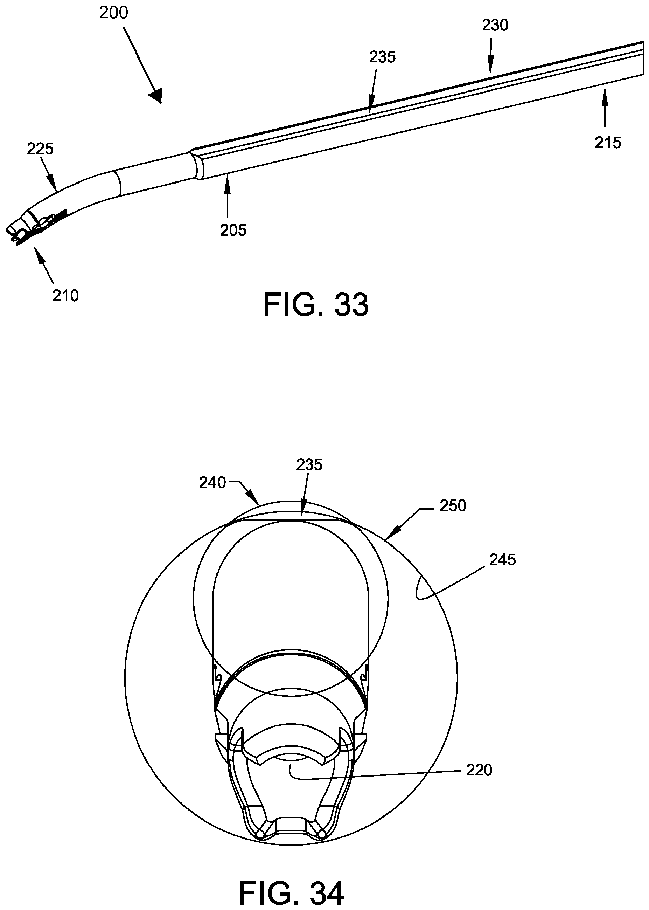

More particularly, and looking now at Figs. 33 and 34, in one preferred form of the invention, there is provided a novel angled drill guide 200 for use in guiding a flexible drill bit (e.g., such as a flexible drill bit discussed above) into target material (e.g., bone). Novel angled drill guide 200 generally comprises an elongated shaft 205 having a distal end 210, a proximal end 215 and a lumen 220 extending therebetween. Preferably elongated shaft 205 has a curved distal portion 225 and a straight proximal portion 230.

In order to allow angled drill guide 200 to be formed with a greater degree of curvature and still pass through the interior lumen of a straight access cannula, at least a portion of straight proximal portion 230 (and, optionally, a portion of curved distal portion 225) is formed with a flat 235 extending therealong, with flat 235 being formed on the same side as the outside of the curve. Flat 235 reduces the effective diameter of elongated shaft 205 so as to minimize interference between the angled drill guide and the side wall of the straight access cannula, thereby allowing angled drill guide 200 to be formed with a greater degree of curvature while still fitting through the straight access cannula with a preferred diameter (e.g., 8 mm inner diameter). See, for example, Fig. 34, which shows how flat 235 on elongated shaft 205 eliminates the area of interference 240 created between angled drill guide 200 and the side wall 245 of a straight access cannula 250.

It will be appreciated that the provision of the flat 235 on elongated shaft 205 can also be used with a curved access cannula so as to eliminate an area of interference between an angled drill guide and the curved access cannula, e.g., where the angled drill guide has an angle of curvature which is greater than the angle of curvature of the curved access cannula.

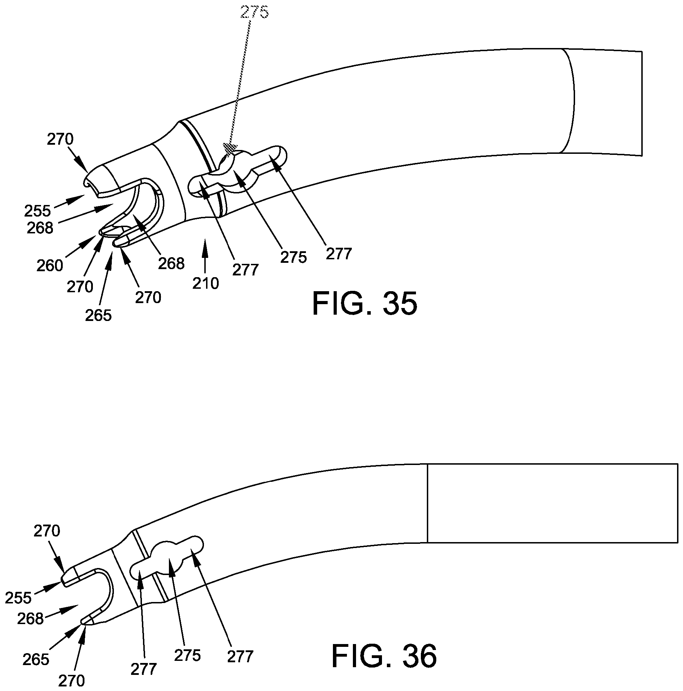

Looking now at Figs. 35 and 36, it will be seen that the distal end 210 of elongated shaft 205 may be formed with three teeth 255, 260, 265 for engaging the surface of the material (e.g., bone) which is to be drilled. Tooth 255 serves to provide a stable support against the material (e.g., bone) which is to be drilled. To this end, tooth 255 is relatively large and is set at the outer perimeter of the curve of angled drill guide 200, thus providing a smooth, continuous surface for a flexible drill bit to ride against as the flexible drill bit passes out the distal end of angled drill guide 200. Specifically, the flexible drill bit may have a tendency to follow the outer perimeter of the curve of angled drill guide 200 when the flexible drill bit is in a flexed state. The cutting edges of the flexible drill bit may catch and/or bear against any irregularities in the surface of angled drill guide 200; therefore, it is preferable to maintain a smooth, uninterrupted surface for the flexible drill bit to bear against. In one preferred form of the invention, tooth 255 extends along approximately 90-180 degrees of the perimeter of the angled drill guide, and preferably along approximately 115 degrees of the perimeter of the angled drill guide. Teeth 260, 265 serve to grip into the material (e.g., bone) which is to be drilled. This is especially significant with an angled drill guide 200, as there are forces imparted on the angled drill guide 200 while drilling into bone (and/or when thereafter implanting an anchor into bone using the angled drill guide) which can tend to make the distal end of the angled drill guide 200 skid along the material (e.g., bone). To this end, teeth 260, 265 are relatively thin and are set at the inner perimeter of the curve of angled drill guide 200. Slots 268 allow the user to view a flexible drill bit exiting the angled drill guide 200. Preferably teeth 255, 260, 265 are radiused at their distal ends (e.g., as shown at 270) so as to facilitate passage of angled drill guide 200 through an access cannula (which may be either straight or curved).

In one preferred form of the invention, angled drill guide 200 also comprises side windows 275 disposed proximal to teeth 255, 260, 265. Preferably side windows 275 have side cuts 277 extending proximally and distally from side windows 275, with side cuts 277 being aligned with the longitudinal axis of angled drill guide 200. Windows 275 allow the user to view a flexible drill bit extending though angled drill guide 200; by providing appropriate markings (not shown) along the shaft of the flexible drill bit, the user can (by aligning those drill bit markings with windows 275) tell the depth to which the flexible drill bit is drilling into the material (e.g., bone), and/or tell the depth to which a bone anchor (being inserted through angled drill guide 200) is inserted into the material (e.g., bone).

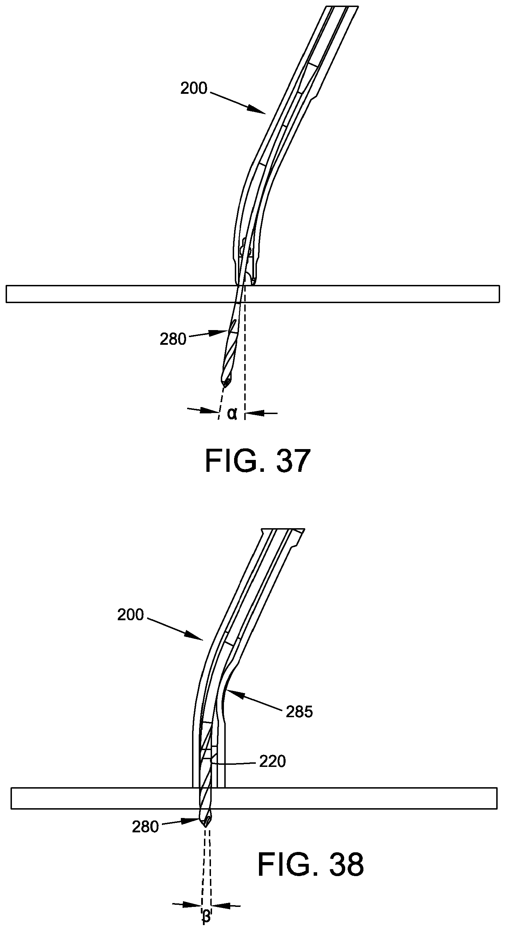

Significantly, where a flexible drill bit passes through an angled drill guide having a curve, the flexible drill bit will tend to bear against the outside of the curve. As a result, when the flexible drill bit exits the distal end of an angled drill guide, the flexible drill bit will tend to exit the distal end of the angled drill guide 200 with an off-angle disposition. See Fig. 37, where a flexible drill bit 280 is shown exiting angled drill guide 200 with offset angle α.

To counteract this effect, and looking now at Fig. 38, angled drill guide 200 may be provided with a dimple 285 in the side wall of the angled drill guide. Dimple 285 is diametrically-opposed to the outside of the curve of angled drill guide 200, and effectively narrows lumen 220. As a result of this construction, when a flexible drill bit is disposed in lumen 220 of the angled drill guide, dimple 285 forces the flexible drill bit into a smaller bend radius that more closely matches the bend radius of the angled drill guide, whereby to re-align the flexible drill bit as it exits the distal end of the angled drill guide 200 and create offset angle β (offset angle β is less than the aforementioned offset angle α). This can be particularly beneficial if the flexible drill bit has a reduced diameter along the length which passes through the curved portion of the angled drill guide 200 (e.g., proximal to the cutting portion as discussed above).

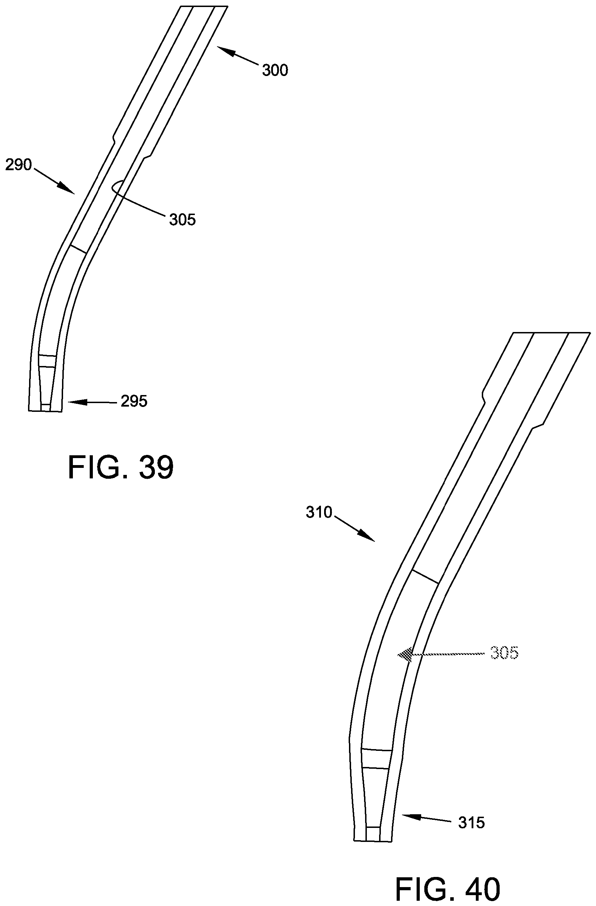

Fig. 39 shows another approach for centering and aligning the flexible drill bit as it exits the distal end of the angled drill guide 290. More particularly, Fig. 39 shows an angled drill guide 290 having a distal end 295, a proximal end 300 and a lumen 305 extending therebetween. In this form of the invention, lumen 305 tapers inwardly (i.e., narrows) at the distal end of angled drill guide 290, whereby to constrain the orientation of a flexible drill bit to a re-centered and re-aligned disposition as it exits the distal end of the angled drill guide. In one preferred form of the invention, lumen 305 narrows so as to provide a bearing structure having a relatively close sliding fit with a flexible drill bit disposed in the angled drill guide, whereby to provide good support for the flexible drill bit as it emerges from the distal end of the angled drill guide. Thus, the flexible drill bit will be more centered with the center axis of the angled drill guide, and will be more angularly aligned with the curvature at the distal end of the angled drill guide.

Fig. 40 shows another angled drill guide 310. Angled drill guide 310 is substantially identical to the angled drill guide 290 shown in Fig. 39, except that with the angled drill guide 310 shown in Fig. 40, distal end 315 of angled drill guide 310 has a tapered outer diameter (as well as a tapered inner diameter) so as to facilitate disposition of the angled drill guide about a drilling site.

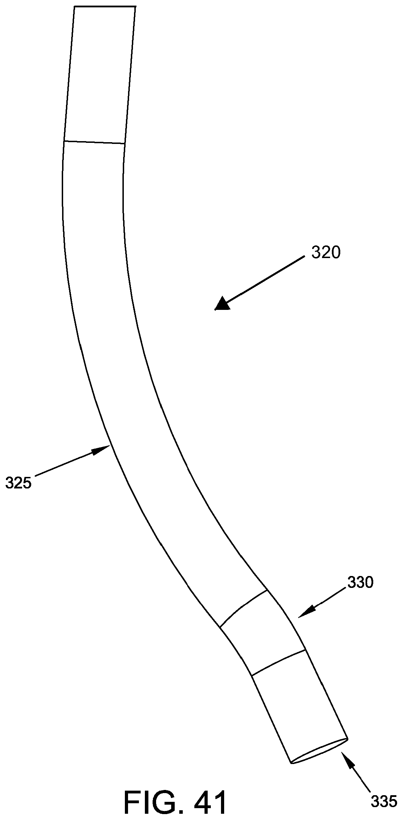

In another form of the present invention, and looking now at Fig. 41, there is shown an angled drill guide 320 which uses the combination of two curves 325, 330 to help center and align the flexible drill bit as it emerges from the distal end 335 of the angled drill guide. More particularly, as noted above, where a flexible drill bit passes through an angled drill guide having a curve, the flexible drill bit will tend to follow the outside of the curve. As a result, when the flexible drill bit exits the distal end of an angled drill guide, the flexible drill bit will tend to exit the distal end of the angled drill guide with an off-centered and mis-aligned disposition. To counteract this effect, the angled drill guide 320 shown in Fig. 41 is formed with two curves 325, 330 - the curve 325 is the primary curve of the angled drill guide, providing the curvature needed for the flexible drill bit to access the drilling site, and the curve 330 is the secondary curve of the angled drill guide, providing the "remedial" curvature used to re-center and re-align the flexible drill bit as it exits the distal end 335 of angled drill guide 320.

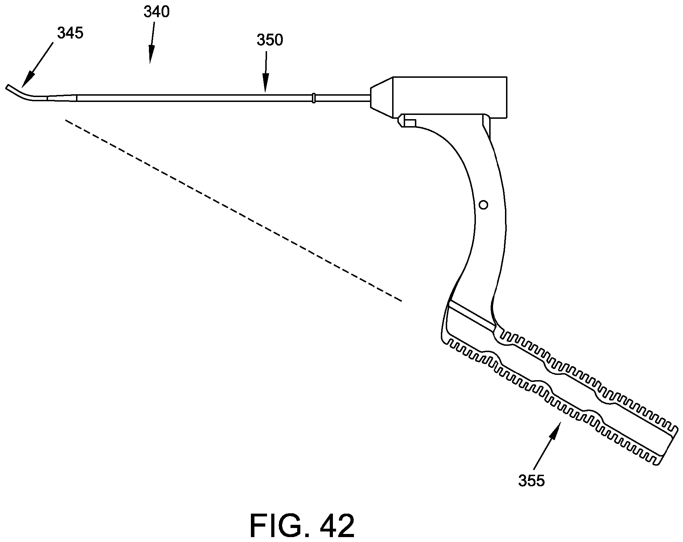

In another form of the present invention, and looking now at Fig. 42, an angled drill guide 340 having a curved distal section 345 and a straight proximal section 350 may be provided with a handle 355 which is offset from the longitudinal axis of the proximal section 350 of the angled drill guide, but which is aligned with the distal section 345 of the angled drill guide, so as to allow the user to better hold the angled drill guide against the material (e.g., bone) which is to be drilled. In essence, by aligning the longitudinal axis of handle 355 with the longitudinal axis of the distal section 345 of the angled drill guide, the user can push the distal end of the angled drill guide directly against the surface of the material (e.g., bone) which is to be drilled, thereby providing more stability during drilling. In other words, the tip of the angled drill guide will be better engaged with the bone and hence less likely to skid along the bone while the hole is being drilled with the angled drill guide and/or an anchor is being placed into the bone hole through the angled drill guide. This is significant, since the forces created during drilling and/or anchor placement through an angled drill guide have a tendency to move the distal end of the angled drill guide relative to the material (e.g., bone) which is being drilled.

The angled drill guide can have a curve of fixed geometry or it is also possible to make an articulating angled drill guide.

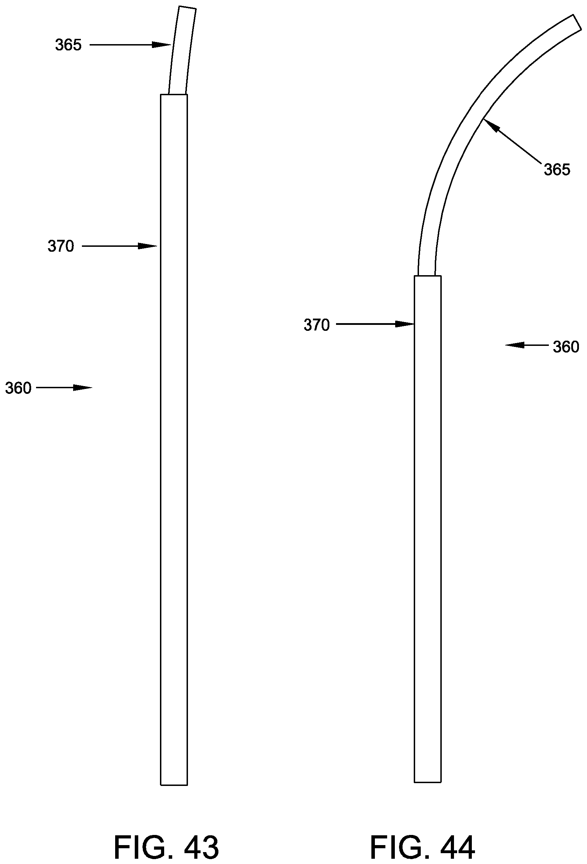

In an example of an articulating angled drill guide, and looking now at Figs. 43 and 44, an angled drill guide 360 may comprise a curved inner sheath 365 for receiving a flexible drill bit (not shown), and a straight outer sheath 370 for overlying some or all of curved inner sheath 365. In this form of the invention, curved inner sheath 365 is in telescoping relation to straight outer sheath 370: retracting curved inner sheath 365 into straight outer sheath 370 causes the curved inner sheath 365 to straighten, while extending curved inner sheath 365 out of straight outer sheath 370 allows the curved inner sheath 365 to curve. Thus, by controlling the disposition of curved inner sheath 365 vis-à-vis straight outer sheath 370, the degree of curvature of the curved inner sheath 365 (and hence the degree of curvature of the angled drill guide as a whole) can be controlled. Curved inner sheath 365 and straight outer sheath 370 are preferably constructed of biocompatible metals; more preferably, curved inner sheath 365 is constructed of superelastic Nitinol and straight outer sheath 370 is constructed of stainless steel.

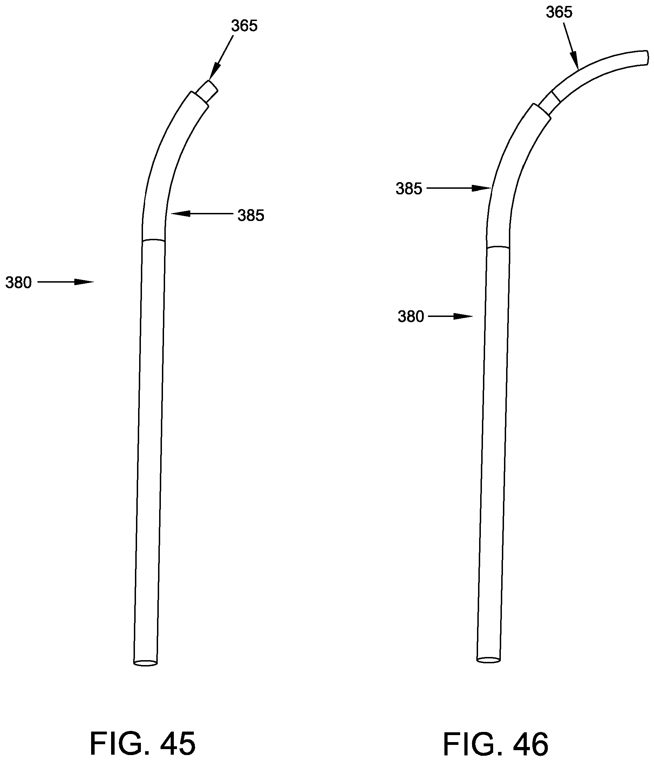

Figs. 45 and 46 show another articulating drill guide 380. More particularly, articulating drill guide 380 is identical to the articulating drill guide 360 shown in Figs. 43 and 44, except that curved inner sheath 365 is slidably disposed in a curved outer sheath 385, wherein curved outer sheath 385 has a lesser degree of curvature than curved inner sheath 365. Again, by controlling the disposition of curved inner sheath 365 vis-à-vis curved outer sheath 370, the degree of curvature of the curved inner sheath 365 (and hence the degree of curvature of the angled drill guide as a whole) can be controlled. Curved inner sheath 365 and curved outer sheath 385 are preferably constructed of biocompatible metal; more preferably, curved inner sheath 365 is constructed of superelastic Nitinol and curved outer sheath 385 is constructed of stainless steel.

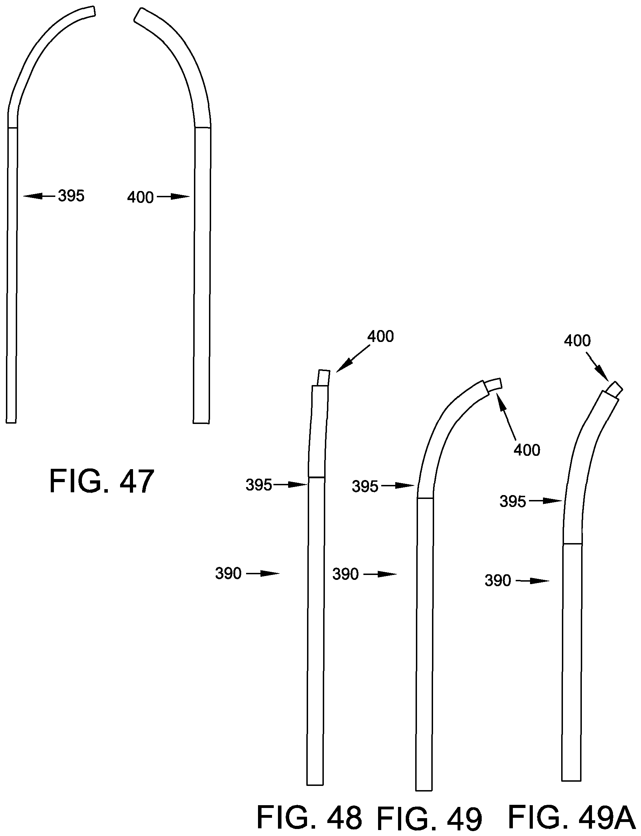

Another articulating angled drill guide 390 is shown in Figs. 47-49. Articulating angled drill guide 390 comprises a curved inner sheath 395 for receiving a flexible drill bit (not shown), and a curved outer sheath 400 for overlying most of curved inner sheath 395. In this form of the invention, rotating curved inner sheath 395 and curved outer sheath 400 relative to one another causes the curves to either (i) counteract one another, whereby to straighten the assembly (see Fig. 48), or (ii) to reinforce one another, whereby to curve the assembly (Fig. 49), or (iii) provide some disposition therebetween (Fig. 49A). Curved inner sheath 395 and curved outer sheath 400 are constructed on biocompatible metal, and preferably of superelastic Nitinol. In order for the curved inner sheath 395 and curved outer sheath 400 to counteract one another (whereby to straighten the assembly), their bending stiffnesses should be similar. However, since the curved outer sheath 400 has a larger diameter, a difference in wall thickness and/or material properties is required in order to achieve a similar bending stiffness. In one example, where both curved inner sheath 395 and curved outer sheath 400 are the same material (e.g., superelastic Nitinol), the curved outer sheath 400 needs to have a thinner wall to achieve a similar bending stiffness to the curved inner sheath 395.

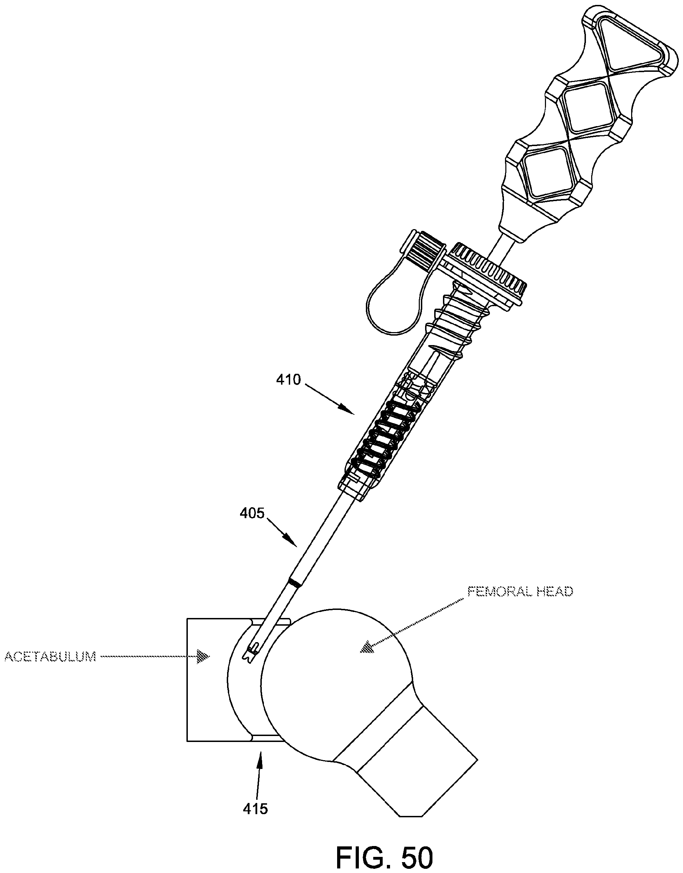

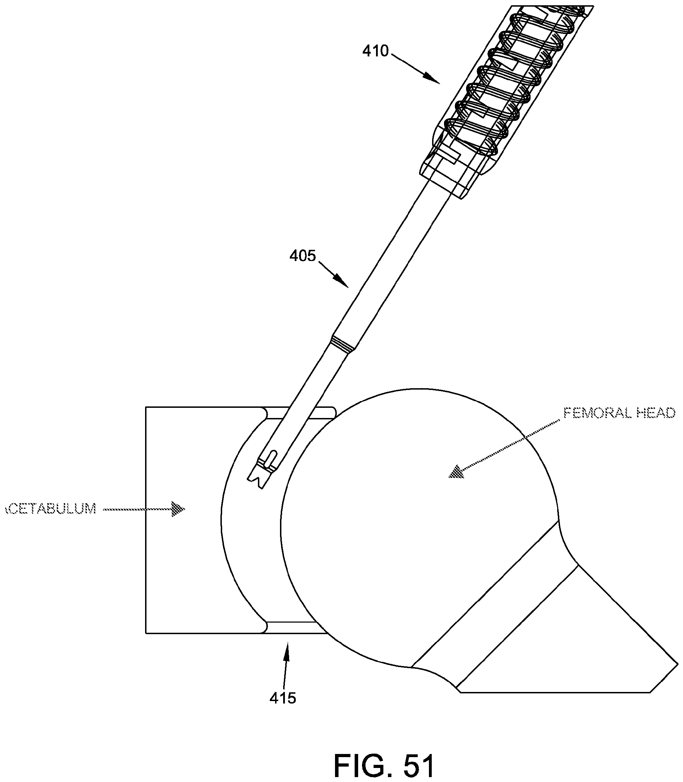

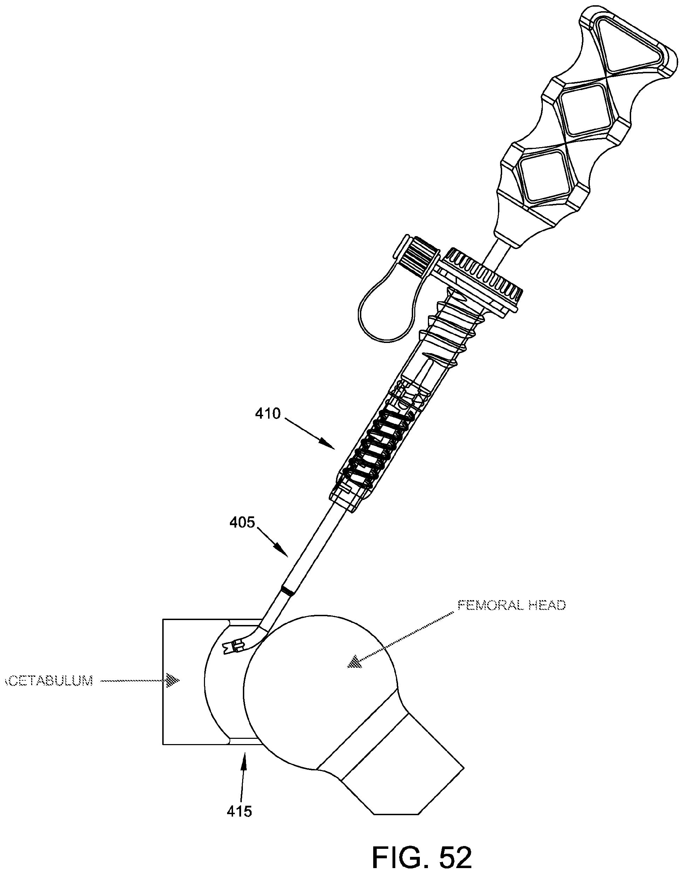

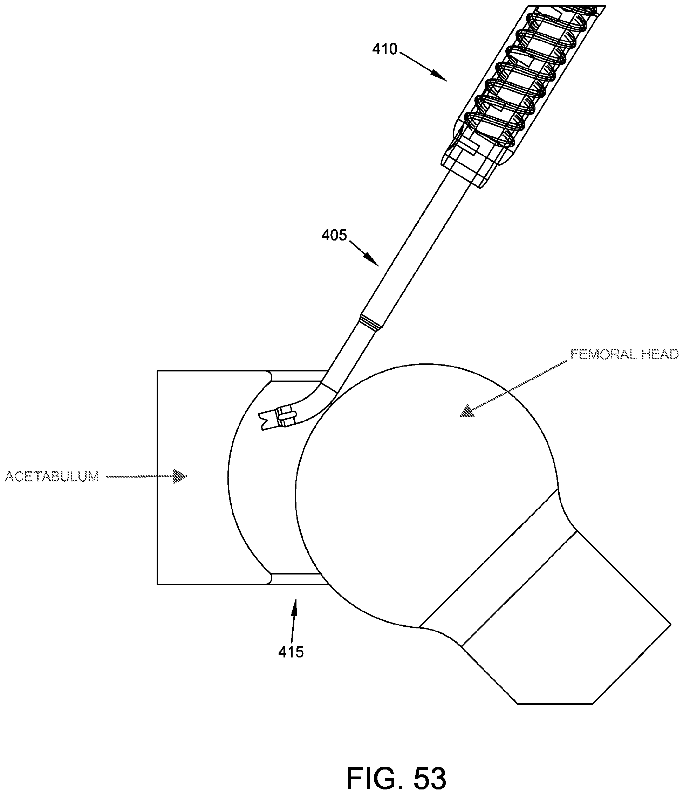

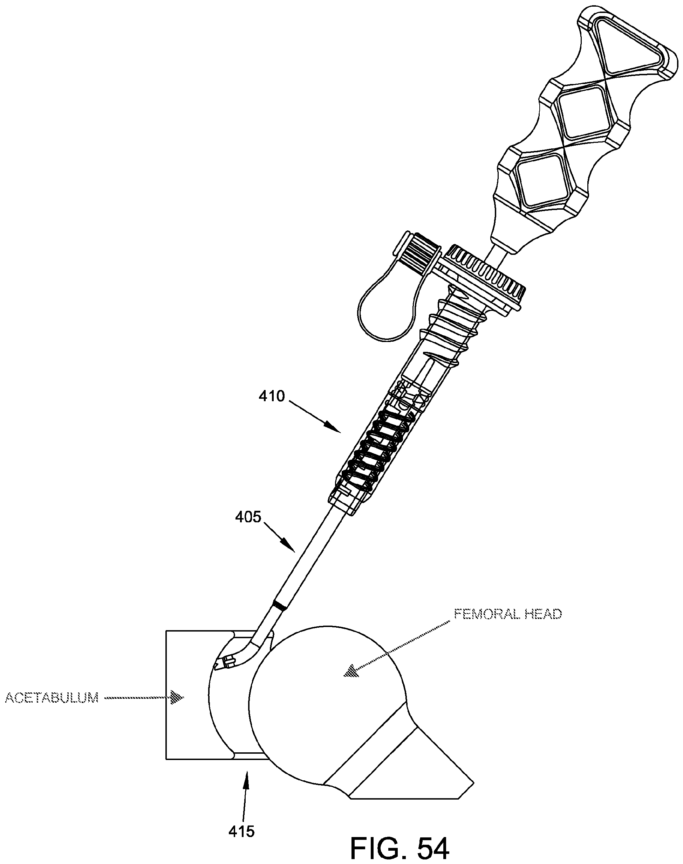

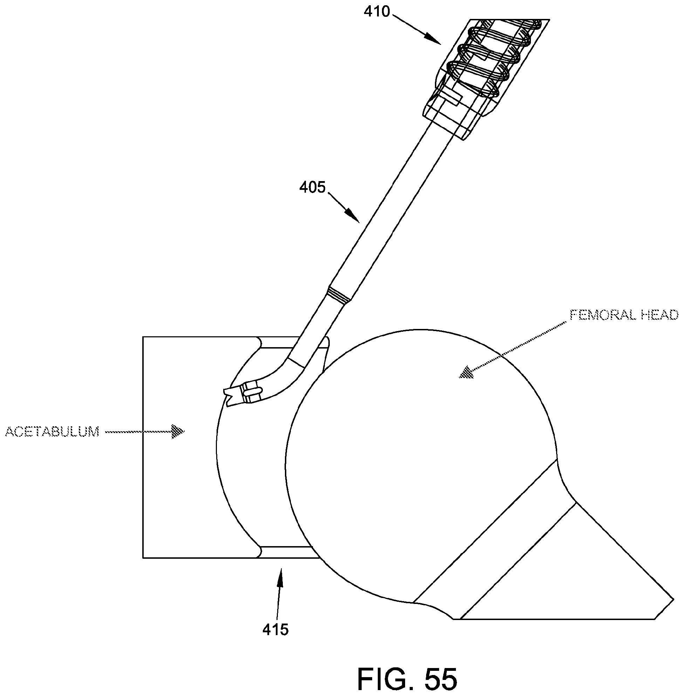

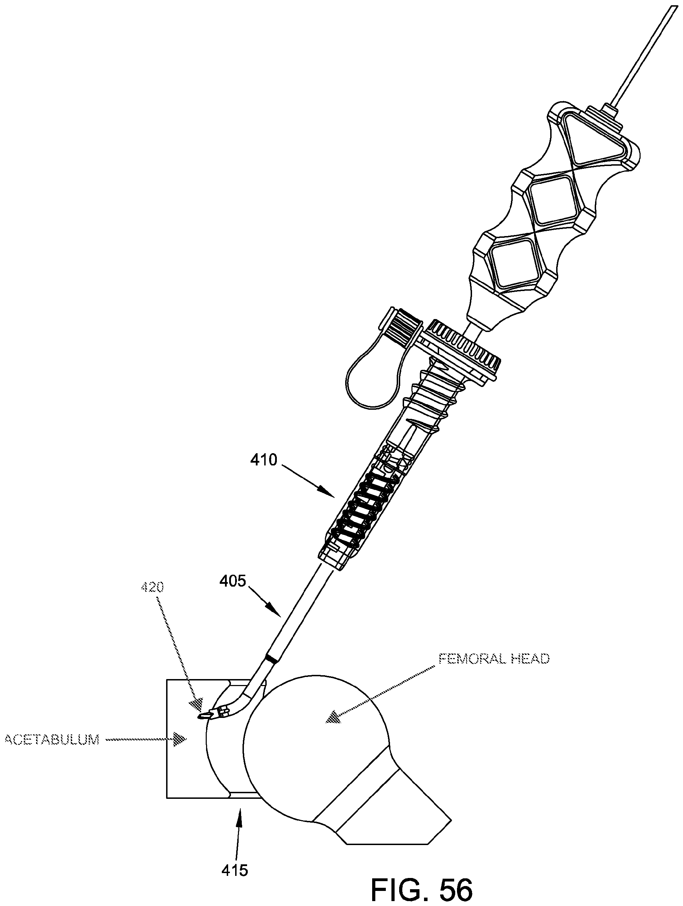

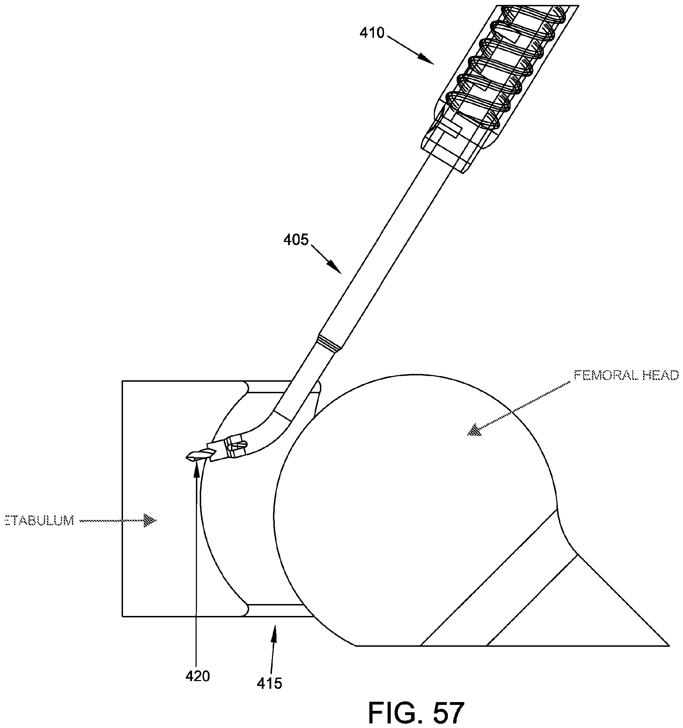

The provision of an articulating angled drill guide and flexible drill bit can be highly advantageous in numerous clinical situations, e.g., when drilling within the interior of a joint. Thus, for example, and looking now at Figs. 50-57, there is shown an articulating angled drill guide 405 extending through an access cannula 410, with the distal end of articulating angled drill guide being disposed within the interior of a joint 415. More particularly, in this form of the invention, articulating angled drill guide 405 may be advanced into the interior of the joint with the articulating angled drill guide in a substantially straight configuration (Figs. 50 and 51). This straight configuration may be helpful in providing a smaller profile by which to pass through the access cannula 410; it may also be helpful in entering a "tight" joint such as the hip joint where the space between the acetabular cup and femoral head is limited. Thereafter, the distal tip of articulating angled drill guide 405 is articulated into a curve so as to address a surface of the joint (Figs. 52 and 53). Next, the distal end of articulating angled drill guide 405 is advanced so that the distal end of the articulating angled drill guide engages the surface which is to be drilled (Figs. 54 and 55). Finally, a flexible drill bit 420 may be advanced through articulating angled drill guide 405 and drilled into the surface of the bone (Figs. 56 and 57).

### Friction-Reducing Flexible Drill Bit

In some situations the curvature of the flexible drill bit within an angled drill guide may be substantial, so that significant friction occurs between the flexible drill bit and the angled drill guide. When operated, the friction between the outer surface of the rotating flexible drill bit and the inner surface of the angled drill guide creates heat which will transfer to the flexible drill bit; a flexible drill bit operating at a higher temperature can have a reduced life. By way of example but not limitation, this can be important where a Nitinol flexible drill bit is operating in a highly stressed condition, so that the life of the Nitinol flexible drill bit is limited and is at or below the intended life of the drill bit to perform its function.

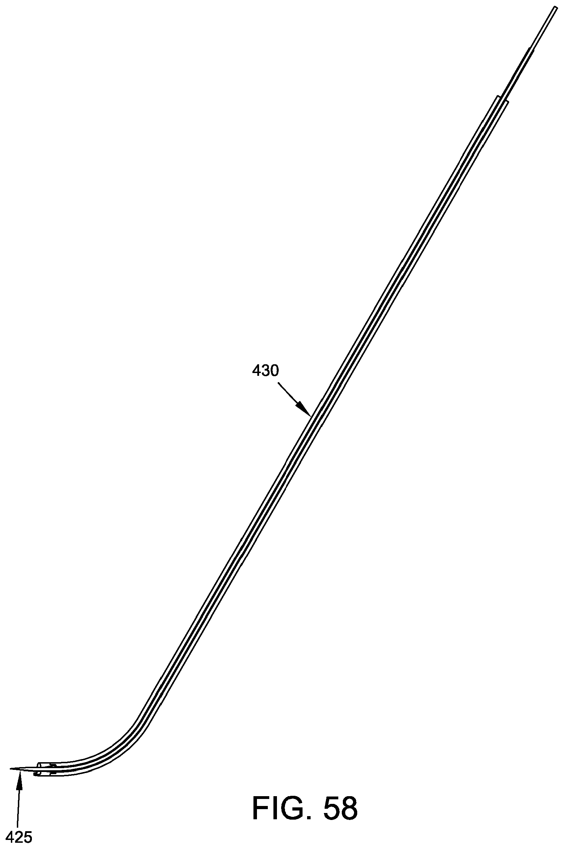

To this end, and looking now at Figs. 58-60, a novel flexible drill bit 425 not according to claim 1 may be provided for use with an angled drill guide 430. Flexible drill bit 425 comprises a Nitinol (or other superelastic material) drill bit 435 having a low-friction coating 440 on its outer surface. In one preferred form of the invention, low-friction coating 440 comprises a polymer (e.g., PTFE) which is heat shrunk onto the outer diameter of Nitinol drill bit 435.

By way of example but not limitation, an angled drill guide was constructed with a distal end having a curve of approximately 60 degrees through an arc of approximately 1 inch radius. A Nitinol drill bit with a diameter of 0.0345 inch was constructed. The Nitinol drill bit was placed into the angled drill guide and operated. After approximately 4 minutes, the drill bit fractured. A second Nitinol drill bit was constructed with a diameter of 0.0345 inch, and a PTFE tube of approximately 0.008 inch thickness was heat shrunk onto the outer diameter of the Nitinol drill bit. The Nitinol drill bit with PTFE coating was placed into the angled drill guide and operated until it fractured, which occurred after approximately 8 minutes. It was found that the Nitinol drill bit with PTFE coating had a significantly longer life than the un-coated Nitinol drill bit in identical test conditions. This was attributed to the fact that the PTFE coating reduced friction between the inner diameter of the angled drill guide and outer diameter of the Nitinol drill bit; this enabled the construction to operate "cooler", which significantly extended the life of a Nitinol drill bit operating in a stressed condition.

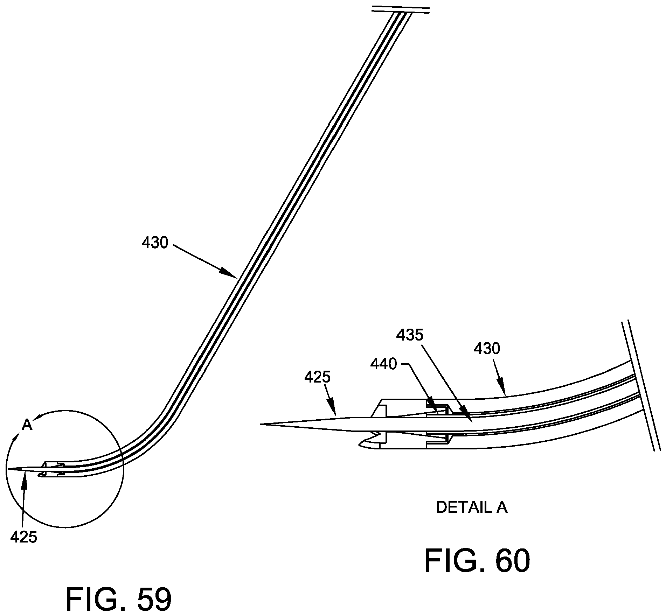

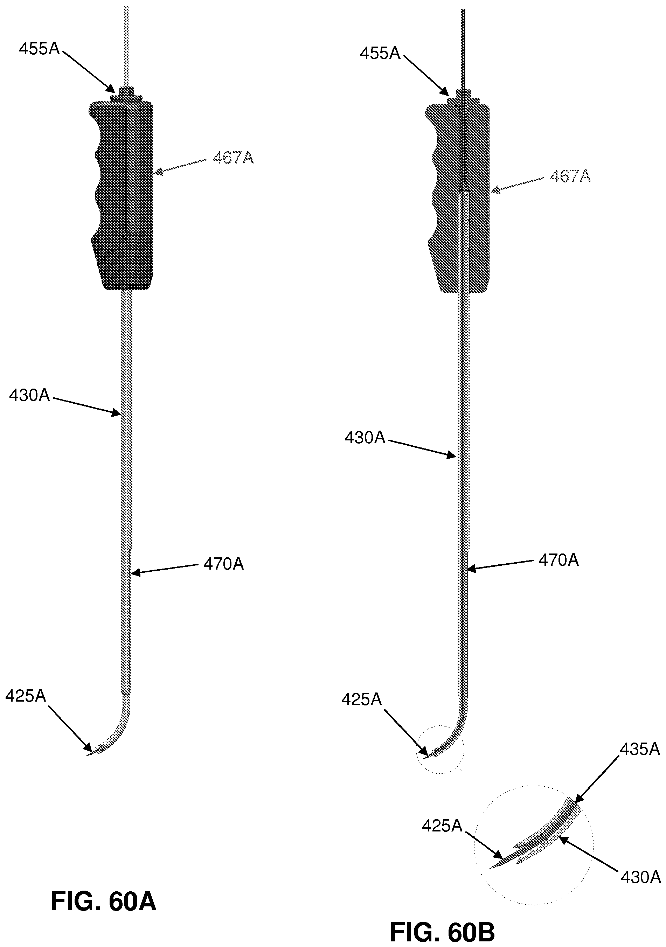

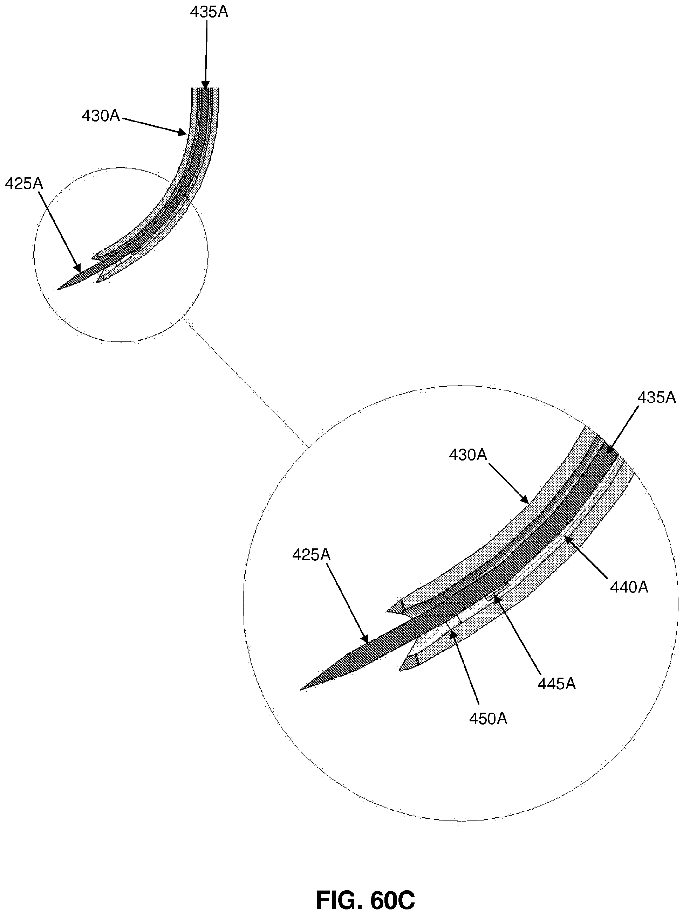

Looking now at Figs. 60A-60E, a flexible drill bit 425A not according to claim 1 may be provided for use with a novel angled drill guide 430A. Flexible drill bit 425A comprises a Nitinol (or other superelastic material) drill bit 435A having a low-friction coating 440A on its outer surface. In one preferred form , low-friction coating 440A comprises a polymer which is heat shrunk onto the outer diameter of Nitinol drill bit 435A. This polymer is preferably FEP (fluorinated ethylene propylene), but it may also be PTFE, Nylon or another low-friction material compatible with the present invention. Low-friction coating 440A reduces the friction generated between flexible drill bit 425A and drill guide 430A, thus reducing the temperature of flexible drill bit 425A; a lower temperature will result in an increased fatigue life (i.e., the lifetime at which it will fail such as by fracturing). Additionally, flexible drill bit 425A may be cooled by the fluid which is typically circulated within the joint space during an arthroscopic procedure. This fluid cooling will act to further extend the life of flexible drill bit 425A.

Flexible drill bit 425A may also comprise a distal stop 445A. Distal stop 445A is formed integral with, or fixed onto, flexible drill bit 425A distal to low-friction coating 440A but proximal to a reduced inner diameter section 450A of angled drill guide 430A. Distal stop 445A has a profile larger than reduced inner diameter section 450A of the angled drill guide 430A. As a result of this construction, in the event that flexible drill bit 425A might fracture at any location proximal to distal stop 445A, distal stop 445A prevents the fractured portion of flexible drill bit 425A from exiting angled drill guide 430A (since distal stop 445A is too large to fit through reduced inner diameter section 450A of angled drill guide 430A), which would be undesirable inasmuch as there would then be a loose component in the joint space which can be difficult to remove.

Flexible drill bit 425A preferably also has a stop 455A which limits the distance which flexible drill bit 425A can extend out of the distal end of angled drill guide 430A. This provides a consistent, controlled drill depth into the bone. Stop 455A is preferably fixed to the proximal portion of the shaft of flexible drill bit 425A (as shown in Figs. 60A and 60B) so as to provide a consistent drill depth hole. However, stop 455A may, alternatively, be movable and/or adjustable along the length of the shaft of flexible drill bit 425A, whereby to allow for a user-adjustable depth to the drill hole. In another aspect of the invention, one or more spacers (e.g., a disc-like spacer, a cylindrical spacer, etc., not shown) can selectively be attached to the distal end of stop 455A, thus reducing the distance flexible drill bit 425A can move distally relative to the angled drill guide 430A, and thus reducing the drill hole depth.

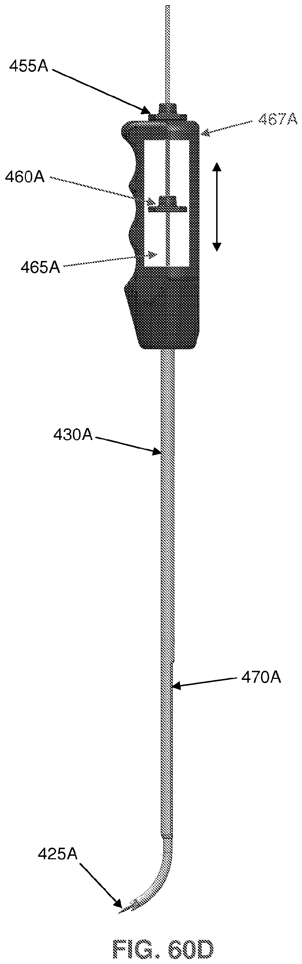

In one aspect of the present invention, and looking now at Fig. 60D, stop 460A is preferably disposed in a cavity 465A formed in the handle 467A of angled drill guide 430A so as to limit travel of the flexible drill bit 425A in the proximal direction, i.e., by engagement of stop 460A with the proximal end of cavity 465A. If desired, with this form of the invention, stop 455A may be omitted and multiple stops 460A may be set on the shaft of flexible drill bit 425A within cavity 465A, with one stop 460A being used to set the limit of distal movement of flexible drill bit 425A and the other stop 460A being used to set the limit of proximal movement of flexible drill bit 425A. Alternatively, a single stop 460A in cavity 465A may be used to set both the distal and proximal limits for flexible drill bit 425A. Note that cavity 465A may be open to the user (e.g., for user adjustment of the position of stop(s) 460A along the shaft of flexible drill bit 425A) or may be closed to the user (in which case the position of stop(s) 460A along the shaft of flexible drill bit 425A is set at the time of assembly).

In one preferred form of the invention, flexible drill bit 425A has a diameter of approximately 0.0345 inch and angled drill guide 430A has a bend radius of approximately 0.825 inch. It has been discovered that this combination of the diameter of flexible drill bit 425A and the radius of curvature of angled drill guide 430A provides the best access into the hip joint while maintaining sufficient fatigue life and torque strength to perform microfracture of an articular defect in the hip (i.e., by forming repeated drill holes into the acetabulum and/or femoral head). The diameter of drill bit 435A can be greater than 0.0345 inch; however, because strains and stresses are greater in a larger diameter drill bit 425A (assuming all other conditions are kept constant), the bend radius of angled drill guide 430A would then need to be larger than 0.825 inch in order to maintain sufficient fatigue life of flexible drill bit 425A. Alternatively, the diameter of flexible drill bit 435A can be smaller than 0.0345 inch; however, a smaller hole may not be a clinically desirable or efficacious and torque strengths may be limited with such a construction.

In a preferred form of the invention, flexible drill bit 435A comprises superelastic Nitinol comprising an oxide layer on its outer surface. Preferably the oxide layer is provided on an intermediate portion of the flexible drill bit, since it has been found that this oxide surface layer provides a surface which has superior fatigue resistance than, for example, a surface that has been mechanically polished, machined or ground to remove the oxide layer. It is believed that these mechanical polishing, machining or grinding processes may leave surface scratches and stress risers (stress concentrations) from which fractures can propagate. However, it is also preferred that the distal end of the flexible drill bit (i.e., at least the portion of the flexible drill bit which extends into the bone) be free of the oxide surface layer, since wear of the oxide surface layer may release undesirable particulates.

Angled drill guide 430A preferably has a cutaway 470A (Figs. 60A, 60B and 60D) on the side of the shaft of angled drill guide 430A which is on the "outside" of the curved distal portion of angled drill guide 430A. This cutaway 470A reduces the profile of the shaft of angled drill guide 430A on the side of the shaft of angled drill guide 430A which typically faces the femoral head during arthroscopic hip surgery (see, for example, Figs. 52-57). This feature provides for more clearance between the femoral head and angled drill guide 430A, thus reducing the chances that angled drill guide 430A will contact and damage the articular surface of the femoral head.

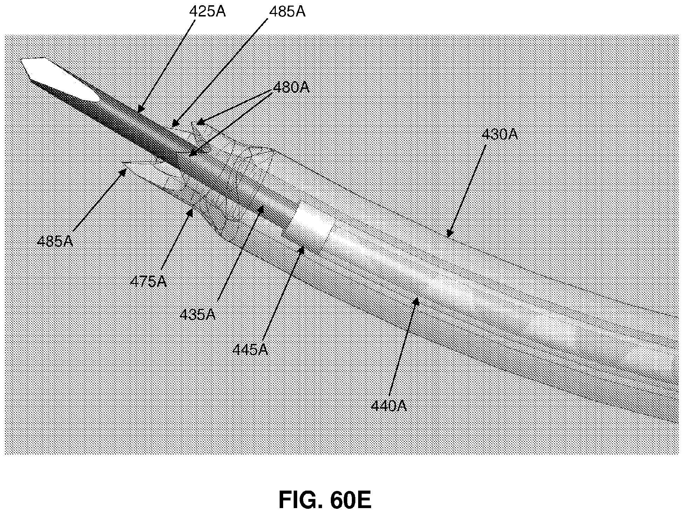

In one form of the present invention, angled drill guide 430A preferably also has a reduced diameter 475A at its distal tip (see Fig. 60E). This allows for better visualization of the target bone site inasmuch as there is less visual obstruction. Reduced diameter 475A also can be a result of forming the reduced inner section 450A in the shaft of angled drill guide 430A (e.g., such as when reduced inner section 450A is produced by crimping angled drill guide 430A inwardly). The distal tip of angled drill guide 430A preferably comprises at least one inner tooth 480A and at least one outer tooth 485A to engage the target bone. Outer tooth 485A preferably extends further distally than inner tooth 480A. This enables both inner tooth 480A and outer tooth 485A to completely engage the target bone when angled drill guide 430A addresses the target site at an angle. This feature will reduce the chances that the tip of angled drill guide 430A will slip on the target bone while flexible drill bit 425A penetrates the bone. In one preferred embodiment, and as shown in Figs. 60A-E, the outer tooth 485A is located on the "outside" of the curved distal portion of angled drill guide 430A, while the inner tooth 480A is located on the "inside" of the curved distal portion of angled drill guide 430A.

By way of example but not limitation, an angled drill guide 430A has been constructed with a distal end having a curve of approximately 60 degrees through an arc of approximately 1 inch radius. A Nitinol drill bit 425A with a diameter of 0.0345 inch was constructed. The Nitinol drill bit 425A was placed into the angled drill guide 430A and operated. After approximately 4 minutes of use, the drill bit fractured. A second Nitinol drill bit was constructed with a diameter of 0.0345 inch, and a PTFE tube 440A of approximately 0.008 inch thickness was heat-shrunk onto the outer diameter of the Nitinol drill bit 425A. The Nitinol drill bit 425A with PTFE coating 440A was placed into the angled drill guide and operated until it fractured, which occurred after approximately 8 minutes of use. It was found that the Nitinol drill bit 425A with PTFE coating 440A had a significantly longer life than the "un-coated" Nitinol drill bit in identical test conditions. This was attributed to the fact that the PTFE coating 440A reduced friction between the inner diameter of the angled drill guide 430A and outer diameter of the Nitinol drill bit 425A; this enabled the construction to operate "cooler", which significantly extended the life of a Nitinol drill bit operating in a stressed condition.

### Additional Subject Matter

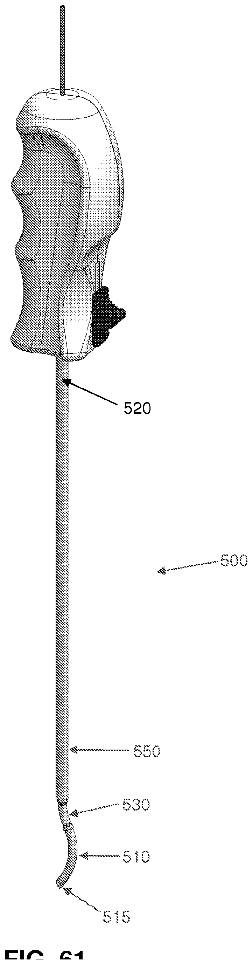

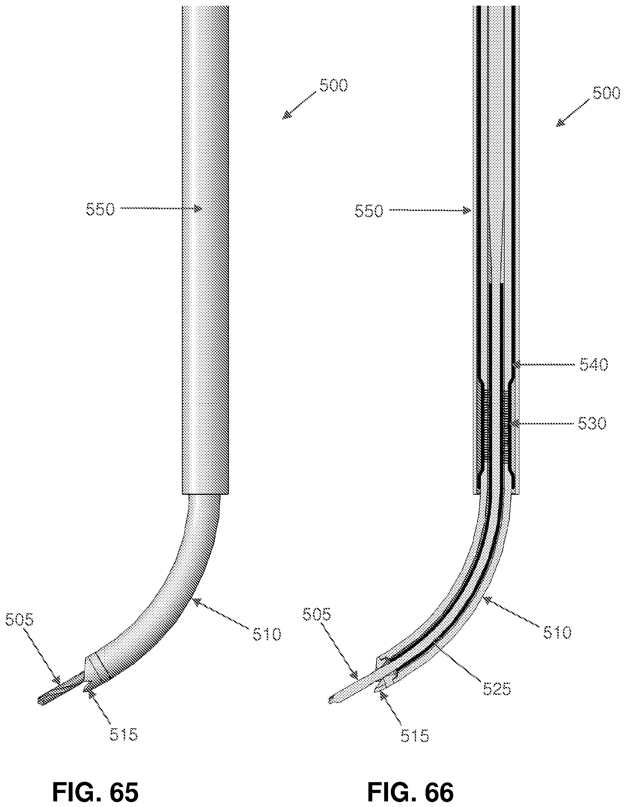

Looking next at Figs. 61-66, there is shown another angled drill guide which may be used with a flexible drill bit.

More particularly, in and looking now at Figs. 61-63, there is provided a novel angled drill guide 500 which may be used to support a flexible drill bit 505. Novel angled drill guide 500 generally comprises an elongated tube 510 having a distal end 515, a proximal end 520 and a lumen 525 extending therebetween. Near its distal end, elongated tube 510 includes a flexible region 530. Flexible region 530 esentially separates elongated tube 510 into a tip portion 535 and a body portion 540. Flexible region 530 is preferably formed by (i) reducing the outer diameter of the side wall of elongated tube 510, or (ii) laser cutting slits in the side wall of elongated tube 510, or (iii) both reducing the outer diameter of the side wall of the elongated tube and laser cutting slits in the side wall of the elongated tube (such as is shown in the figures). An FEP sleeve 545 is preferably heat-shrunk over flexible region 530 so as to provide flexible region 530 with a smooth outer profile without interfering with its flexibility.

A telescoping outer tube 550 is positioned coaxially over elongated tube 510. Telescoping outer tube 550 is selectively advanceable over flexible region 530 of elongated tube 510 so as to selectively stabilize distal portion 535 of elongated tube 510 relative to body portion 540 of elongated tube 510.

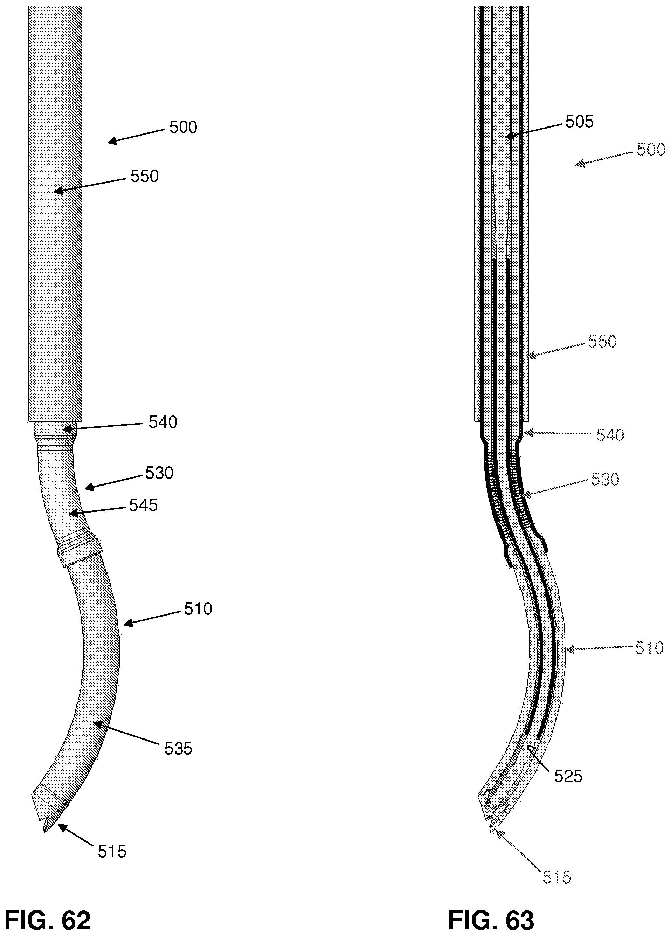

Flexible drill bit 505 is preferably pre-loaded into novel angled drill guide 500 prior to use, with flexible drill bit 505 positioned so that the leading tip of the flexible drill bit resides just inside lumen 525 of elongated tube 510 (see Fig. 63). Such pre-loading of flexible drill bit 505 into angled drill guide 500 may be effected in the operating room or at the time of manufacture or assembly.

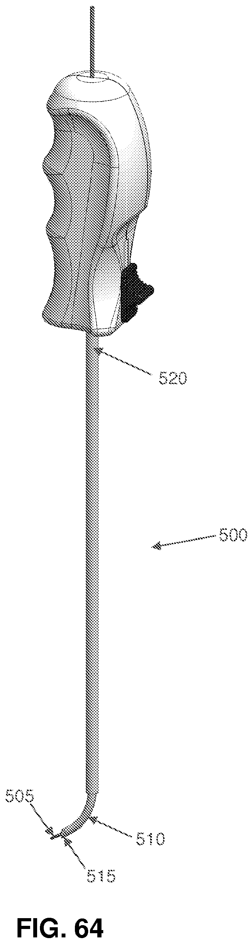

In use, telescoping outer tube 550 is initially retracted to the position shown in Figs. 61-63 so that tip portion 535 of elongated tube 510 is able to flex relative to body portion 540 of elongated tube 510. This allows angled drill guide 500 to pass down the surgical corridor leading to the arthroscopic site (e.g., through a straight access cannula). Then telescoping outer tube 550 is advanced forward, extending over flexible region 530 of elongate tube 510, whereby to stabilize tip portion 535 of elongated tube 510 relative to body portion 540 of elongated tube 510 (Figs. 64-66), with tip portion 535 extending at an angle to the longitudinal axis of body portion 540. With tip portion 535 of elongated tube 510 so stabilized, flexible drill bit 505 is advanced out the distal end of the angled drill guide, whereby to form a hole in a target bone. Then, when the desired hole has been formed, flexible drill bit 505 may be withdrawn and angled drill guide 500 may be used for other purposes, e.g., to deliver a bone anchor into the hole formed in the target bone. When angled drill guide 500 is to be withdrawn, telescoping outer tube 550 is pulled back, thereby restoring flexibility to flexible region 530 of elongated tube 510, and then elongated tube 510 is retracted back along the surgical corridor to the surface of the skin.

### Modifications

While the present invention has been described in terms of certain exemplary preferred embodiments, it will be readily understood and appreciated by those skilled in the art that it is not so limited, and that many additions, deletions and modifications may be made to the preferred embodiments discussed herein without departing from the scope of the invention as defined in the appended claims.

## Claims

1. Surgical apparatus for drilling a hole in bone, comprising:
- an angled drill guide (35, 200, 290, 310, 320, 340, 360, 380, 390, 405) comprising
- a curved distal section (225, 345, 365, 395), a less-curved proximal section (230, 350, 370, 385, 400), and a lumen (50, 220, 305) extending therebetween; and
- a flexible drill bit (5) disposed within the lumen of the angled drill guide, the flexible drill bit comprising:
- a proximal shaft portion (10) for connecting to a source of turning;
- a distal cutting tip portion (20) for boring into a bone; and
- an intermediate shaft portion (15) extending between the proximal shaft portion and the distal cutting tip portion, wherein the diameter of the intermediate shaft portion (15) is smaller than the diameter of the distal cutting tip portion (20), and further wherein the diameter of the intermediate shaft portion (15) is smaller than the diameter of the proximal shaft portion (10), the intermediate shaft portion being **characterized by** (i) sufficient longitudinal flexibility so as to permit the flexible drill bit to be passed along a curve, and (ii) sufficient torsional strength to permit the flexible drill bit to bore into the bone.

2. Surgical apparatus according to claim 1, **characterized by** a handle (355) mounted to the angled drill guide so that the handle is offset from the longitudinal axis of the less-curved proximal section (350) of the angled drill guide and aligned with the curved distal section (345) of the angled drill guide, whereby to allow the user to push the distal end of the angled drill guide directly against the outer surface of the material which is to be drilled.

3. Surgical apparatus according to claim 1, **characterized by** a stop (455A) fixed to the proximal shaft portion of the flexible drill bit, or movable and/or adjustable along the length of the shaft of the drill bit, so as to limit the distance the flexible drill bit can extend out of angled drill guide.

4. Surgical apparatus according to claim 1, wherein the less-curved proximal section (230) comprises a flat (235) extending therealong for reducing the effective diameter of the less-curved proximal section so as to minimize interference between the angled drill guide and the side wall of an access cannula.

5. Surgical apparatus according to claim 1, wherein the curved distal section and less-curved proximal section form part of an elongated shaft (205), wherein the curved distal section forms an inside of a curve and an outside of the curve, wherein the curved distal section terminates in a distal end of the elongated shaft, wherein the distal end comprises a perimeter extending around the distal end from the inside of the curve of the curved distal section to the outside of the curve of the curved distal section, wherein the distal end of the elongated shaft comprises first, second and third teeth (255, 260, 265) extending distally therefrom, wherein the first tooth (255) is set along the perimeter at the outside of the curve of the curved distal section, and wherein the second tooth and third tooth (260, 265) are set along the perimeter at the inside of the curve of the curved distal section.

6. Surgical apparatus according to claim 5, wherein the curved distal section comprises at least one window (275) extending through a side wall thereof, wherein the at least one window comprises a circular opening, a distal side cut (277) extending distally from the circular opening and a proximal side cut (277) extending proximally from the circular opening, wherein the distal side cut and the proximal side cut align with a longitudinal axis of the angled drill guide and each have a width which is less than the width of the circular opening, so as to allow a user to view a flexible drill bit disposed within the lumen of the angled drill guide.

7. Surgical apparatus according to claim 1, wherein the curved distal section comprises a dimple (285) for effectively narrowing the lumen of the angled drill guide opposite to the curve of the angled drill guide, whereby to angularly re-align a flexible drill bit exiting the distal section of the angled drill guide.

8. Surgical apparatus according to claim 1, wherein the lumen tapers inwardly at the distal end of the angled drill guide so as to re-center a flexible drill bit exiting the distal end of the angled drill guide.

9. Surgical apparatus according to claim 1, wherein the curved distal section comprises compound curves so as to re-align a flexible drill bit exiting the distal section of the angled drill guide.

10. Surgical apparatus according to claim 1, **characterized by** an articulating angled drill guide comprising a curved inner sheath (365) and a straight outer sheath (370) for overlaying some or all of curved inner sheath, wherein the curved inner sheath is telescopically received within the straight outer sheath.

11. Surgical apparatus according to claim 1, **characterized by** an articulating angled drill guide comprising a curved inner sheath (365, 395) and a curved outer sheath (385, 400), wherein the curved outer sheath has a lesser degree of curvature than curved inner sheath, wherein the curved inner sheath is slidably received within the curved outer sheath.

12. Surgical apparatus according to claim 1, comprising a friction-reducing flexible drill bit, **characterized by** a flexible drill bit of a superelastic material and having a low-friction coating formed thereon.

13. Surgical apparatus according to claim 1, wherein the lumen narrows at a first location along the curved distal section, wherein the flexible drill bit comprises a diametrical enlargement at a second location proximal to the first location.

14. Surgical apparatus according to claim 1, **characterized by** an angled drill guide, wherein
- the curved distal section, the less-curved proximal section, and an intermediate flexible section extending between the curved distal section and the less-curved proximal section are formed by an inner component, wherein the lumen extends through the curved distal section, the intermediate flexible section and the less-curved proximal section;
further comprising:
- an outer component comprising a distal end, a proximal end and a lumen extending therebetween;
- the inner component being receivable within the lumen of the outer component and the inner component and outer component being selectively movable such that (i) the intermediate flexible section of the inner component is received within the lumen of the outer component, and (ii) the intermediate flexible section of the inner component is disposed distal to the outer component.

15. Surgical apparatus according to claim 6, wherein the flexible drill bit comprises a marking on an outer surface of the flexible drill bit, and further wherein when the marking on the outer surface of the flexible drill bit is directly aligned with the circular opening of the at least one window of the angled drill guide, a user is informed of the depth to which the flexible drill bit is drilling in the bone.

16. Surgical apparatus according to any of claims 1-15, wherein the outer diameter of the intermediate shaft portion (15) is approximately 20%-40%, such as approximately 25%, smaller than the diameter of the proximal shaft portion (10).

17. Surgical apparatus according to any of claims 1-16, wherein the outer diameter of the intermediate shaft portion (15) is approximately 5-25%, such as approximately 15%, smaller than the outer diameter of the distal cutting tip portion (20).

18. Surgical apparatus according to any of claims 1-17, wherein the length of the distal cutting tip portion (20) is approximately six times greater than the diameter of the distal cutting tip portion.

19. Surgical apparatus according to any of claims 1-18, wherein a transition area (30) is formed between the intermediate shaft portion (15) and the distal cutting tip portion (20), wherein the transition area tapers from the distal cutting tip portion to the intermediate shaft portion.

20. Surgical apparatus according to any of claims 1-19, wherein a transition area (25) is formed between the intermediate shaft portion (15) and the proximal shaft portion (10).

21. Surgical apparatus according to any of claims 1-20, wherein the curvature of the angled drill guide, the outer diameter of the intermediate shaft portion (15) and the inner diameter of the lumen are chosen such that the distal cutting tip portion (20) is not coaxial with the lumen as the distal cutting tip portion emerges from the distal end of the angled drill guide.

22. Surgical apparatus according to any of claims 1-21, wherein a gap is formed between a side wall of the lumen of the angled drill guide and the intermediate shaft portion (15) of the flexible drill bit (5) when the intermediate shaft portion of the flexible drill bit is received in the lumen of the angled drill guide, and further wherein the gap is sized so that when the distal end of the angled drill guide is set perpendicularly to the material, the distal cutting tip portion (20) of the flexible drill bit (5) will enter the material at a non-perpendicular angle.

## Patentansprüche

1. Chirurgische Einrichtung zum Bohren eines Loches im Knochen, umfassend:
- eine abgewinkelte Bohrerführung (35, 200, 290, 310, 320, 340, 360, 380, 390, 405), umfassend
- einen gekrümmten distalen Abschnitt (225, 345, 365, 395), einen weniger gekrümmten proximalen Abschnitt (230, 350, 370, 385, 400) und ein Lumen (50, 220, 305), das sich dazwischen erstreckt; und
- einen flexiblen Bohrmeißel (5), der in dem Lumen der abgewinkelten Bohrführung angeordnet ist, wobei der flexible Bohrmeißel Folgendes umfasst:
- einen proximalen Schaftabschnitt (10) zur Verbindung mit einer Drehquelle;
- einen distalen Schneidspitzenabschnitt (20) zum Bohren in einen Knochen;
- einen Zwischenschaftabschnitt (15), der sich zwischen dem proximalen Schaftabschnitt und dem distalen Schneidspitzenabschnitt erstreckt, wobei der Durchmesser des Zwischenschaftabschnitts (15) kleiner ist als der Durchmesser des distalen Schneidspitzenabschnitts (20) und wobei weiter der Durchmesser des Zwischenschaftabschnitts (15) kleiner ist als der Durchmesser des proximalen Schaftabschnitts (10), wobei der Zwischenschaftabschnitt **gekennzeichnet ist durch** (i) eine ausreichende Längsflexibilität, sodass der flexible Bohrmeißel entlang einer Kurve geführt werden kann, und (ii) eine ausreichende Torsionsfestigkeit, damit sich der flexible Bohrmeißel in den Knochen bohren kann.

2. Chirurgische Einrichtung nach Anspruch 1, **gekennzeichnet durch** einen Handgriff (355), der so an der abgewinkelten Bohrerführung angebracht ist, dass der Handgriff von der Längsachse des weniger gekrümmten proximalen Abschnitts (350) der abgewinkelten Bohrerführung versetzt ist und mit dem gekrümmten distalen Abschnitt (345) der abgewinkelten Bohrerführung ausgerichtet ist, wodurch es dem Benutzer ermöglicht wird, das distale Ende der abgewinkelten Bohrerführung direkt gegen die Außenfläche des zu bohrenden Materials zu drücken.

3. Chirurgische Einrichtung nach Anspruch 1, **gekennzeichnet durch** einen Anschlag (455A), der am proximalen Schaftabschnitt des flexiblen Bohrmeißels befestigt oder entlang der Länge des Schaftes des Bohrmeißels beweglich und/oder einstellbar ist, um den Abstand zu begrenzen, den der flexible Bohrmeißel aus der abgewinkelten Bohrerführung herausragen kann.

4. Chirurgische Einrichtung nach Anspruch 1, wobei der weniger gekrümmte proximale Abschnitt (230) eine sich längs desselben erstreckende Abflachung (235) zur Verringerung des effektiven Durchmessers des weniger gekrümmten proximalen Abschnitts umfasst, um die Interferenz zwischen der abgewinkelten Bohrführung und der Seitenwand einer Zugangskanüle zu minimieren.

5. Chirurgische Einrichtung nach Anspruch 1, wobei der gekrümmte distale Abschnitt und der weniger gekrümmte proximale Abschnitt einen Teil eines länglichen Schafts (205) bilden, wobei der gekrümmte distale Abschnitt eine Innenseite einer Krümmung und eine Außenseite der Krümmung bildet, wobei der gekrümmte distale Abschnitt in einem distalen Ende des länglichen Schafts endet, wobei das distale Ende einen Umfang umfasst, der sich um das distale Ende von der Innenseite der Krümmung des gekrümmten distalen Abschnitts zur Außenseite der Krümmung des gekrümmten distalen Abschnitts erstreckt, wobei das distale Ende des länglichen Schafts einen ersten, einen zweiten und einen dritten Zahn (255, 260, 265) umfasst, die sich distal davon erstrecken, wobei der erste Zahn (255) entlang des Umfangs an der Außenseite der Krümmung des gekrümmten distalen Abschnitts angeordnet ist, und wobei der zweite Zahn und der dritte Zahn (260, 265) entlang des Umfangs an der Innenseite der Krümmung des gekrümmten distalen Abschnitts angeordnet sind.

6. Chirurgische Einrichtung nach Anspruch 5, wobei der gekrümmte distale Abschnitt mindestens ein Fenster (275) umfasst, das sich durch eine Seitenwand davon erstreckt, wobei das mindestens eine Fenster eine kreisförmige Öffnung, einen distalen Seitenschnitt (277), der sich distal von der kreisförmigen Öffnung erstreckt, und einen proximalen Seitenschnitt (277) umfasst, der sich proximal von der kreisförmigen Öffnung erstreckt, wobei der distale Seiteneinschnitt und der proximale Seiteneinschnitt mit einer Längsachse der abgewinkelten Bohrerführung fluchten und jeweils eine Breite aufweisen, die geringer ist als die Breite der kreisförmigen Öffnung, um es einem Benutzer zu ermöglichen, einen flexiblen Bohrmeißel zu betrachten, der innerhalb des Lumens der abgewinkelten Bohrerführung angeordnet ist.

7. Chirurgische Einrichtung nach Anspruch 1, wobei der gekrümmte distale Abschnitt eine Vertiefung (285) umfasst, um das Lumen der abgewinkelten Bohrführung gegenüber der Krümmung der abgewinkelten Bohrführung wirksam zu verengen, wodurch ein flexibler Bohrmeißel, der aus dem distalen Abschnitt der abgewinkelten Bohrführung austritt, winkelmäßig neu ausgerichtet wird.

8. Chirurgische Einrichtung nach Anspruch 1, wobei sich das Lumen am distalen Ende der abgewinkelten Bohrführung nach innen verjüngt, um einen flexiblen Bohrmeißel, der aus dem distalen Ende der abgewinkelten Bohrführung austritt, neu zu zentrieren.

9. Chirurgische Einrichtung nach Anspruch 1, wobei der gekrümmte distale Abschnitt zusammengesetzte Kurven umfasst, um einen flexiblen Bohrmeißel, der aus dem distalen Abschnitt der abgewinkelten Bohrerführung austritt, neu auszurichten.

10. Chirurgische Einrichtung nach Anspruch 1, **gekennzeichnet durch** eine gelenkige, abgewinkelte Bohrerführung, umfassend eine gekrümmte Innenhülse (365) und eine gerade Außenhülse (370) zum Überlagern eines Teils oder der gesamten gekrümmten Innenhülse, wobei die gekrümmte Innenhülse teleskopisch innerhalb der geraden Außenhülse aufgenommen ist.

11. Chirurgische Einrichtung nach Anspruch 1, **gekennzeichnet durch** eine gelenkige, abgewinkelte Bohrerführung, umfassend eine gekrümmte Innenhülse (365, 395) und eine gekrümmte Außenhülse (385, 400), wobei die gekrümmte Außenhülse einen geringeren Krümmungsgrad als die gekrümmte Innenhülse aufweist, wobei die gekrümmte Innenhülse gleitend in der gekrümmten Außenhülse aufgenommen ist.

12. Chirurgische Einrichtung nach Anspruch 1, umfassend einen reibungsmindernden flexiblen Bohrmeißel, **gekennzeichnet durch** einen flexiblen Bohrmeißel aus einem superelastischen Material und mit einer darauf ausgebildeten reibungsarmen Beschichtung.

13. Chirurgische Einrichtung nach Anspruch 1, wobei sich das Lumen an einer ersten Stelle entlang des gekrümmten distalen Abschnitts verengt, wobei der flexible Bohrmeißel eine diametrale Vergrößerung an einer zweiten Stelle proximal zur ersten Stelle umfasst.

14. Chirurgische Einrichtung nach Anspruch 1, **gekennzeichnet durch** eine abgewinkelte Bohrerführung, wobei
- der gekrümmte distale Abschnitt, der weniger gekrümmte proximale Abschnitt und ein flexibler Zwischenabschnitt, der sich zwischen dem gekrümmten distalen Abschnitt und dem weniger gekrümmten proximalen Abschnitt erstreckt, durch ein inneres Bauteil gebildet werden, wobei sich das Lumen durch den gekrümmten distalen Abschnitt, den flexiblen Zwischenabschnitt und den weniger gekrümmten proximalen Abschnitt erstreckt;
ferner umfassend:
- ein äußeres Bauteil, das ein distales Ende, ein proximales Ende und ein sich dazwischen erstreckendes Lumen umfasst;
- wobei das innere Bauteil in dem Lumen des äußeren Bauteils aufnehmbar ist und das innere Bauteil und das äußere Bauteil selektiv beweglich sind, sodass (i) der flexible Zwischenabschnitt des inneren Bauteils in dem Lumen des äußeren Bauteils aufgenommen ist und (ii) der flexible Zwischenabschnitt des inneren Bauteils distal zu dem äußeren Bauteil angeordnet ist.

15. Chirurgische Einrichtung nach Anspruch 6, wobei der flexible Bohrmeißel eine Markierung auf einer Außenfläche des flexiblen Bohrmeißels umfasst, und wobei ferner, wenn die Markierung auf der Außenfläche des flexiblen Bohrmeißels direkt mit der kreisförmigen Öffnung des mindestens einen Fensters der abgewinkelten Bohrführung ausgerichtet ist, ein Benutzer über die Tiefe informiert wird, bis zu der sich der flexible Bohrmeißel in den Knochen bohrt.

16. Chirurgische Einrichtung nach einem der Ansprüche 1 bis 15, wobei der Außendurchmesser des Zwischenschaftabschnitts (15) etwa 20 bis 40 %, beispielsweise etwa 25 %, kleiner ist als der Durchmesser des proximalen Schaftabschnitts (10).

17. Chirurgische Einrichtung nach einem der Ansprüche 1 bis 16, wobei der Außendurchmesser des Zwischenschaftabschnitts (15) etwa 5 bis 25 %, beispielsweise etwa 15 %, kleiner ist als der Außendurchmesser des distalen Schneidspitzenabschnitts (20).

18. Chirurgische Einrichtung nach einem der Ansprüche 1 bis 17, wobei die Länge des distalen Schneidspitzenabschnitts (20) etwa sechsmal größer ist als der Durchmesser des distalen Schneidspitzenabschnitts.

19. Chirurgische Einrichtung nach einem der Ansprüche 1 bis 18, wobei zwischen dem Zwischenschaftabschnitt (15) und dem distalen Schneidspitzenabschnitt (20) ein Übergangsbereich (30) ausgebildet ist, wobei sich der Übergangsbereich vom distalen Schneidspitzenabschnitt zum Zwischenschaftabschnitt verjüngt.

20. Chirurgische Einrichtung nach einem der Ansprüche 1 bis 19, wobei zwischen dem Zwischenschaftabschnitt (15) und dem proximalen Schaftabschnitt (10) ein Übergangsbereich (25) ausgebildet ist.

21. Chirurgische Einrichtung nach einem der Ansprüche 1 bis 20, wobei die Krümmung der abgewinkelten Bohrerführung, der Außendurchmesser des Zwischenschaftabschnitts (15) und der Innendurchmesser des Lumens so gewählt sind, dass der distale Schneidspitzenabschnitt (20) nicht koaxial mit dem Lumen ist, wenn der distale Schneidspitzenabschnitt aus dem distalen Ende der abgewinkelten Bohrerführung austritt.

22. Chirurgische Einrichtung nach einem der Ansprüche 1 bis 21, wobei ein Spalt zwischen einer Seitenwand des Lumens der abgewinkelten Bohrführung und dem Zwischenschaftabschnitt (15) des flexiblen Bohrmeißels (5) gebildet wird, wenn der Zwischenschaftabschnitt des flexiblen Bohrmeißels in dem Lumen der abgewinkelten Bohrführung aufgenommen wird, und wobei der Spalt so bemessen ist, dass, wenn das distale Ende der abgewinkelten Bohrführung senkrecht auf das Material eingestellt ist, der distale Schneidspitzenabschnitt (20) des flexiblen Bohrmeißels (5) in einem nicht senkrechten Winkel in das Material eintritt.

## Revendications

1. Appareil chirurgical pour percer un trou dans un os, comprenant :
- un guide de perçage oblique (35, 200, 290, 310, 320, 340, 360, 380, 390, 405) comprenant
- une section distale courbée (225, 345, 365, 395), une section proximale moins courbée (230, 350, 370, 385, 400), et une lumière (50, 220, 305) s'étendant entre celles-ci ; et
- un trépan flexible (5) disposé à l'intérieur de la lumière du guide de perçage oblique, le trépan flexible comprenant :
- une partie formant tige proximale (10) pour la liaison à une source de rotation ;
- une partie formant bout de coupe distale (20) pour le perçage dans un os ;
et
- une partie formant tige intermédiaire (15) s'étendant entre la partie formant tige proximale et la partie formant bout de coupe distale, dans lequel le diamètre de la partie formant tige intermédiaire (15) est plus petit que le diamètre de la partie formant bout de coupe distale (20), et en outre dans lequel le diamètre de la partie formant tige intermédiaire (15) est plus petit que le diamètre de la partie formant tige proximale (10), la partie formant tige intermédiaire étant **caractérisée par** (i) une flexibilité longitudinale suffisante de manière à permettre au trépan flexible d'être amenée à passer le long d'une courbe, et (ii) une résistance à la torsion suffisante pour permettre au trépan flexible d'effectuer le perçage dans l'os.

2. Appareil chirurgical selon la revendication 1, **caractérisé par** une poignée (355) montée sur le guide de perçage oblique de sorte que la poignée est décalée de l'axe longitudinal de la section proximale moins courbée (350) du guide de perçage oblique et alignée sur la section distale courbée (345) du guide de perçage oblique, de manière à permettre à l'utilisateur de pousser l'extrémité distale du guide de perçage oblique directement contre la surface extérieure du matériau qui est à percer.

3. Appareil chirurgical selon la revendication 1, **caractérisé par** une butée (455A) fixée à la partie formant tige proximale du trépan flexible, ou mobile et/ou réglable le long de la longueur de la tige du trépan, de manière à limiter la distance sur laquelle le trépan flexible peut s'étendre hors du guide de perçage oblique.

4. Appareil chirurgical selon la revendication 1, dans lequel la section proximale moins courbée (230) comprend une facette (235) s'étendant le long de celle-ci pour réduire le diamètre effectif de la section proximale moins courbée de manière à minimiser l'interférence entre le guide de perçage oblique et la paroi latérale d'une canule d'accès.

5. Appareil chirurgical selon la revendication 1, dans lequel les section distale courbée et section proximale moins courbée font partie d'une tige allongée (205), dans lequel la section distale courbée forme un intérieur d'une courbe et un extérieur de la courbe, dans lequel la section distale courbée termine dans une extrémité distale de la tige allongée, dans lequel l'extrémité distale comprend un périmètre s'étendant autour de l'extrémité distale à partir de l'intérieur de la courbe de la section distale courbée vers l'extérieur de la courbe de la section distale courbée, dans lequel l'extrémité distale de la tige allongée comprend des première, deuxième et troisième dents (255, 260, 265) s'étendant de manière distale à partir de celle-ci, dans lequel la première dent (255) est positionnée le long du périmètre au niveau de l'extérieur de la courbe de la section distale courbée, et dans lequel les deuxième dent et troisième dent (260, 265) sont positionnées le long du périmètre au niveau de l'intérieur de la courbe de la section distale courbée.

6. Appareil chirurgical selon la revendication 5, dans lequel la section distale courbée comprend au moins une fenêtre (275) s'étendant à travers une paroi latérale de celle-ci, dans lequel l'au moins une fenêtre comprend une ouverture circulaire, une découpe latérale distale (277) s'étendant de manière distale à partir de l'ouverture circulaire et une découpe latérale proximale (277) s'étendant de manière proximale à partir de l'ouverture circulaire, dans lequel la découpe latérale distale et la découpe latérale proximale s'alignent sur un axe longitudinal du guide de perçage oblique et présentent chacune une largeur qui est inférieure à la largeur de l'ouverture circulaire, de manière à permettre à un utilisateur de visualiser un trépan flexible disposé dans la lumière du guide de perçage oblique.

7. Appareil chirurgical selon la revendication 1, dans lequel la section distale courbée comprend une alvéole (285) pour rétrécir efficacement la lumière du guide de perçage oblique à l'opposé de la courbe du guide de perçage oblique, de manière à réaligner angulairement un trépan flexible quittant la section distale du guide de perçage oblique.

8. Appareil chirurgical selon la revendication 1, dans lequel la lumière s'effile vers l'intérieur à l'extrémité distale du guide de perçage oblique de manière à recentrer un trépan flexible quittant l'extrémité distale du guide de perçage oblique.

9. Appareil chirurgical selon la revendication 1, dans lequel la section distale courbée comprend des courbes composées de manière à réaligner un trépan flexible quittant la section distale du guide de perçage oblique.

10. Appareil chirurgical selon la revendication 1, **caractérisé par** un guide de perçage oblique articulé comprenant une gaine intérieure courbée (365) et une gaine extérieure rectiligne (370) pour recouvrir une partie ou la totalité de la gaine intérieure courbée, dans lequel la gaine intérieure courbée est reçue de manière télescopique dans la gaine extérieure rectiligne.

11. Appareil chirurgical selon la revendication 1, **caractérisé par** un guide de perçage oblique articulé comprenant une gaine intérieure courbée (365, 395) et une gaine extérieure courbée (385, 400), dans lequel la gaine extérieure courbée présente un degré de courbure plus petit que la gaine intérieure courbée, dans lequel la gaine intérieure courbée est reçue de manière coulissante dans la gaine extérieure courbée.

12. Appareil chirurgical selon la revendication 1, comprenant un trépan flexible à réduction de friction, **caractérisé par** un trépan flexible composé d'un matériau superélastique et présentant un revêtement à frottement réduit formé sur celui-ci.

13. Appareil chirurgical selon la revendication 1, dans lequel la lumière rétrécit au niveau d'un premier emplacement le long de la section distale courbée, dans lequel le trépan flexible comprend un élargissement diamétral au niveau d'un deuxième emplacement proximal par rapport au premier emplacement.

14. Appareil chirurgical selon la revendication 1, **caractérisé par** un guide de perçage oblique, dans lequel
- la section distale courbée, la section proximale moins courbée, et une section flexible intermédiaire s'étendant entre la section distale courbée et la section proximale moins courbée sont formées par un composant intérieur, dans lequel la lumière s'étend à travers la section distale courbée, la section flexible intermédiaire et la section proximale moins courbée ;
comprenant en outre :
- un composant extérieur comprenant une extrémité distale, une extrémité proximale et une lumière s'étendant entre celles-ci ;
- le composant intérieur pouvant être reçu dans la lumière du composant extérieur et les composant intérieur et composant extérieur étant mobiles sélectivement de telle sorte que (i) la section flexible intermédiaire du composant intérieur est reçue dans la lumière du composant extérieur, et (ii) la section flexible intermédiaire du composant intérieur est disposée de manière distale par rapport au composant extérieur.

15. Appareil chirurgical selon la revendication 6, dans lequel le trépan flexible comprend un marquage sur une surface extérieure du trépan flexible, et en outre dans lequel lorsque le marquage sur la surface extérieure du trépan flexible est directement aligné sur l'ouverture circulaire de l'au moins une fenêtre du guide de perçage oblique, un utilisateur est informé de la profondeur à laquelle le trépan flexible effectue un perçage dans l'os.

16. Appareil chirurgical selon l'une quelconque des revendications 1-15, dans lequel le diamètre extérieur de la partie formant tige intermédiaire (15) est approximativement 20 %-40 %, par exemple approximativement 25 % plus petit que le diamètre de la partie formant tige proximale (10).

17. Appareil chirurgical selon l'une quelconque des revendications 1-16, dans lequel le diamètre extérieur de la partie formant tige intermédiaire (15) est approximativement 5-25 %, par exemple approximativement 15 % plus petit que le diamètre extérieur de la partie formant bout de coupe distale (20).

18. Appareil chirurgical selon l'une quelconque des revendications 1-17, dans lequel la longueur de la partie formant bout de coupe distale (20) est approximativement six fois plus grand que le diamètre de la partie formant bout de coupe distale.

19. Appareil chirurgical selon l'une quelconque des revendications 1-18, dans lequel une zone de transition (30) est formée entre la partie formant tige intermédiaire (15) et la partie formant bout de coupe distale (20), dans lequel la zone de transition s'effile à partir de la partie formant bout de coupe distale vers la partie formant tige intermédiaire.

20. Appareil chirurgical selon l'une quelconque des revendications 1-19, dans lequel une zone de transition (25) est formée entre la partie formant tige intermédiaire (15) et la partie formant tige proximale (10) .

21. Appareil chirurgical selon l'une quelconque des revendications 1-20, dans lequel la courbure du guide de perçage oblique, le diamètre extérieur de la partie formant tige intermédiaire (15) et le diamètre intérieur de la lumière sont choisis de telle sorte que la partie formant bout de coupe distale (20) n'est pas coaxiale avec la lumière à mesure que la partie formant bout de coupe distale émerge de l'extrémité distale du guide de perçage oblique.

22. Appareil chirurgical selon l'une quelconque des revendications 1-21, dans lequel un espace est formé entre une paroi latérale de la lumière du guide de perçage oblique et la partie formant tige intermédiaire (15) du trépan flexible (5) lorsque la partie formant tige intermédiaire du trépan flexible est reçue dans la lumière du guide de perçage oblique, et en outre dans lequel l'espace est dimensionné de sorte que lorsque l'extrémité distale du guide de perçage oblique est positionnée perpendiculairement au matériau, la partie formant bout de coupe distale (20) du trépan flexible (5) pénétrera dans le matériau à un angle non perpendiculaire.
